(19) Europäisches Patentamt
European Patent Office
Office européen des brevets

(11) **EP 2 716 652 A1**

(12) **EUROPEAN PATENT APPLICATION**
published in accordance with Art. 153(4) EPC

(43) Date of publication:
**09.04.2014 Bulletin 2014/15**

(21) Application number: **12774359.9**

(22) Date of filing: **16.04.2012**

(51) Int Cl.:
***C07K 7/08*** *(2006.01)*
***C12N 15/11*** *(2006.01)*
***C12N 15/63*** *(2006.01)*
***A61K 38/10*** *(2006.01)*
***A61P 35/00*** *(2006.01)*
***A61P 1/16*** *(2006.01)*
***A61P 3/04*** *(2006.01)*

(86) International application number:
**PCT/CN2012/074107**

(87) International publication number:
**WO 2012/142930 (26.10.2012 Gazette 2012/43)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.04.2011 CN 201110102020**

(71) Applicant: **Tianjin Toptech Bio-science&technology Co., Ltd.**
**Tianjin 300457 (CN)**

(72) Inventors:
- **ZHANG, Xiaodong**
  **Tianjin 300071 (CN)**
- **YE, Lihong**
  **Tianjin 300071 (CN)**

(74) Representative: **Engelhard, Markus**
**Boehmert & Boehmert**
**Pettenkoferstrasse 20-22**
**80336 München (DE)**

# (54) ANTI-FATTY ACID SYNTHASE POLYPEPTIDE AND USE THEREOF

(57) The present invention relates to a polypeptide that is capable of inhibiting transcription and expression of fatty acid synthase (FAS) and the polynucleotides encoding therefor, as well as the use thereof. Specifically, the present invention relates to a polypeptide that can inhibit the transcription and expression of FAS at the molecular level, the cellular level and *in vivo,* and can therefore prevent the overexpression of FAS. Said polypeptide and related peptidomimetics, including functional fragments or functional varieties thereof, and the genes encoding therefor, can be widely used in preventing and treating tumors such as liver cancer, and diseases closely related to the metabolism of fatty acid synthase, such as fatty liver and obesity.

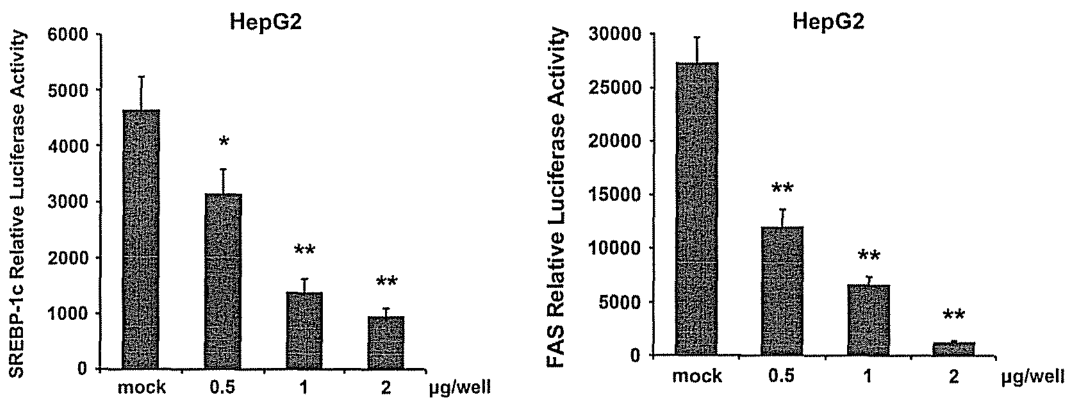


Fig.2

EP 2 716 652 A1

SEQUENCE LISTING

<110> TIANJIN TOPTECH BIO_SCIENCE & TECHNOLOGY CO., LTD

<120> ANTI-FATTY ACID SYNTHASE POLYPEPTIDE AND USE THEREOF

<130> SPI111834-50

<160> 16

<170> PatentIn version 3.3

<210> 1
<211> 18
<212> PRT
<213> Polypeptide

<400> 1

```
Gly Gly Cys Arg His Lys Leu Val Cys Ala Pro Ala Pro Cys Asn Phe
1               5                   10                  15


Phe Thr
```

<210> 2
<211> 19
<212> PRT
<213> Polypeptide

<400> 2

```
Val Gly Gly Cys Arg His Lys Leu Val Cys Ala Pro Ala Pro Cys Asn
1               5                   10                  15


Phe Phe Thr
```

<210> 3
<211> 19
<212> PRT
<213> Polypeptide

<400> 3

```
Ile Gly Gly Cys Arg His Lys Leu Val Cys Ala Pro Ala Pro Cys Asn
1               5                   10                  15


Phe Phe Thr
```

<210> 4
<211> 18
<212> PRT
<213> Polypeptide

<400> 4

Gly Gly Cys Lys His Lys Leu Val Cys Ala Pro Ala Pro Cys Asn Phe
1 5 10 15

Phe Thr

<210> 5
<211> 18
<212> PRT
<213> Polypeptide

<400> 5

Gly Gly Cys Arg Arg Lys Leu Val Cys Ala Pro Ala Pro Cys Asn Phe
1 5 10 15

Phe Thr

<210> 6
<211> 18
<212> PRT
<213> Polypeptide

<400> 6

Gly Gly Cys Arg His His Leu Val Cys Ala Pro Ala Pro Cys Asn Phe
1 5 10 15

Phe Thr

<210> 7
<211> 54
<212> DNA
<213> Artificial Sequence

<400> 7
ggaggctgta ggcataaatt ggtctgcgca ccagcaccat gcaacttttt cacc 54

<210> 8
<211> 57
<212> DNA
<213> Artificial Sequence

<400> 8
gtcggaggct gtaggcataa attggtctgc gcaccagcac catgcaactt tttcacc 57

<210> 9
<211> 57
<212> DNA
<213> Artificial Sequence

<400> 9

atcggaggct gtaggcataa attggtctgc gcaccagcac catgcaactt tttcacc 57

<210> 10
<211> 54
<212> DNA
<213> Artificial Sequence

<400> 10
ggaggctgta aacataaatt ggtctgcgca ccagcaccat gcaacttttt cacc 54

<210> 11
<211> 54
<212> DNA
<213> Artificial Sequence

<400> 11
ggaggctgtc atcataaatt ggtctgcgca ccagcaccat gcaacttttt cacc 54

<210> 12
<211> 54
<212> DNA
<213> Artificial Sequence

<400> 12
ggaggctgta ggcatcattt ggtctgcgca ccagcaccat gcaacttttt cacc 54

<210> 13
<211> 66
<212> DNA
<213> Forward DNA Oligo

<400> 13
aattcatggg aggctgtagg cataaattgg tctgcgcacc agcaccatgc aactttttca 60

cctgac 66

<210> 14
<211> 66
<212> DNA
<213> Reverse DNA Oligo

<400> 14
tcgagtcagg tgaaaaagtt gcatggtgct ggtgcgcaga ccaatttatg cctacagcct 60

cccatg 66

<210> 15
<211> 20
<212> DNA
<213> Forward DNA Oligo

<400> 15
taatacgact cactataggg 20

<210> 16
<211> 18

```
<212>  DNA
<213>  Reverse DNA Oligo

<400>  16
tagaaggcac agtcgagg                                                18
```

## Description

### Related Applications

This application claims the benefit of Chinese Patent Application No. 201110102020.6, filed on April 22, 2011, and entitled "Anti-Fatty Acid Synthase Polypeptide and Use Thereof," which is incorporated herein by reference in its entirety.

### Technical Field

The present invention relates to the technology of polypeptide medicine, and especially relates to the polypeptides of anti-fatty acid synthase and polynucleotides encoding these polypeptides, and methods of use.

### Background

Fatty acid synthase (Fatty acid syntheses, FAS) is a key enzyme in the process of endogenous fatty acid synthesis in living organism, and it produces long-chain fatty acids by catalyzing acetyl coenzyme A and malonyl coenzyme A. FAS comprises 7 functional domains which are acetyltransferase (AT), malonyl transferase (MT), beta-ketoacyl synthase (KS), beta-ketoacyl reductase (KR), beta-hydroxyacyl dehydratase (HD), enoyl reductase (ER) and thioesterase (TE). FAS is closely linked to obesity, and has a high expression in adipose tissue. Meanwhile, FAS in human liver has an even higher expression, and the capacity of synthesizing fatty acid in liver is 8 to 9 times higher than that in adipose tissue. Therefore, FAS is closely linked to the formation of fatty liver. "Fatty liver" refers to a lesion that excessively accumulates fat in the liver cells due to various causes. In addition, traditional theory states that the metabolism of fatty acid synthesis was once considered largely an pathway for storing anabolic-energy, but a great deal of researches have shown that the FAS expression level in tumor tissue such as breast cancer, liver cancer, prostate cancer, ovarian cancer, colorectal cancer and endometrial cancer is much higher than that in normal tissue, and inhibiting the activity of FAS in above-mentioned tumor tissue significantly suppresses tumor growth. Therefore, FAS is increasingly becoming the new drug target for treatment of these diseases.

Therefore, the study of FAS inhibitors for suppression of endogenous fatty acid biosynthesis is of great significance in effectively controlling the occurrence and development of diseases such as cancer, fatty liver, obesity and related metabolic syndrome. Past studies have suggested that specific small molecule inhibitors of FAS can reduce the synthesis of fatty acids by inhibiting the FAS. Fatty acid synthesis is blocked, resulting in increase of the concentration of the substrate malonyl coenzyme A thereof, which may act directly on feeding center in the hypothalamus, inhibiting the secretion of neuropeptide Y that can stimulate the feeding, and thereby leading to feeding suppression. In addition, FAS inhibitors can also improve non-insulin-dependent diabetes, reduce high blood pressure, coronary thrombosis and other symptoms of complications of obesity, reduce incidences thereof. Currently, chemical substances such as sulfur esters C75 structural analogs, butyrolactone and butyrolactam compounds, urea compounds, polyphenolic plant extracts and hydroxy quinoline were known to have the function on inhibiting fatty acid biosynthesis enzymes. However, these compound inhibitors have strong poisonous side effects as common chemical drugs, and some chemicals are unstable, therefore they are constrained in the clinical application.

### Summary of Invention

The present invention relates to an anti-fatty acid synthase(FAS) polypeptide, , which has a role in inhibiting the transcription and expression of FAS, and further inhibiting the FAS expression *in vitro* and *in vivo.* The polypeptides and related peptidomimetic, including functional fragments or functional variants thereof, as well as the genes encoding said peptides and peptidomimetics, as well as functional fragments or variant thereof, can be widely used for preventing and treating the occurrence and development of tumors, fatty liver and obesity, including inhibition of the growth and development in hepatoma cell.

In one aspect, the present invention provides isolated polypeptides or peptidomimetics, which comprise the amino acid sequence as shown in SEQ ID NO: 1, or the functional fragment and functional variant thereof; said polypeptides or peptidomimetics are capable of inhibiting the transcription and expression of FAS, and can be widely used for preventing and treating the occurrence and development of tumors, fatty liver and obesity, including inhibition of the growth and development of liver cancer.

In some embodiments of present invention, said polypeptides or peptidomimetics, or the functional fragments and functional variants thereof, comprise amino acid sequence that have at least 80% identity to the amino acid sequence shown in SEQ ID NO: 1, or at least 90% identity, or at least 95% identity, or even higher.

In another embodiments of present invention, said polypeptides or peptidomimetics comprising amino acid

sequence obtained from conservative substitution of one or plural amino acids of SEQ ID NO:1, or addition or deletion one or plural amino acids of SEQ ID NO:1 in either terminus or both termini thereof. Preferably, said polypeptides or peptidomimetics comprising amino acid sequence obtained from conservative substitution of one amino acid of SEQ ID NO:1, or addition or deletion one amino acid of SEQ ID NO:1 in either terminus thereof. More preferably, said polypeptides or peptidomimetics comprise any one of the amino acid sequences shown in SEQ ID NOs: 1-6.

In another aspect, the present invention provides isolated polynucleotides; said polynucleotides encode the polypeptides comprising the amino acid sequence as shown in SEQ ID NO: 1, or the functional fragments and variants thereof; alternatively, said polynucleotides are complementary to the polynucleotides encoding the polypeptides comprising the amino acid sequence of SEQ ID NO: 1 or the functional fragments and variants thereof, or can hybridize with them under stringent hybridization condition. Preferably, said polypeptides or peptidomimetics comprising amino acid sequence obtained from conservative substitution of one or plural amino acids of SEQ ID NO:1, or addition or deletion one or plural amino acids of SEQ ID NO:1 in either terminus or both termini thereof. More preferably, said polypeptides or peptidomimetics comprising amino acid sequence obtained from conservative substitution of one amino acid of SEQ ID NO:1, or addition or deletion one amino acid of SEQ ID NO:1 in either terminus thereof. Even more preferably, said functional fragments and functional variants of said polypeptides, comprise any one of the amino acid sequence shown in SEQ ID NOs: 2-6.

In another aspect, the present invention also provides a recombinant expression vector containing an exogenous polynucleotide that encodes the amino acid sequence shown in SEQ ID NO: 1, or the functional fragments and variants thereof; alternatively, the vector can contain a polynucleotide that is either complementary to the aforesaid exogenous polynucleotide encoding the amino acid sequence shown in SEQ ID NO: 1 or capable of hybridizing with the aforesaid exogenous polynucleotide under stringent hybridization condition.

In addition, the present invention also provides usage of saidpolypeptide or peptidomimetics, the polynucleotides, and the recombinant expression vectors in the manufacture of medicines for treating and preventing tumors, fatty liver and obesity. In a specific embodiment, said medicine can inhibit the growth of hepatoma cells *in vivo* and *in vitro,* therefore preventing and treating liver cancer. Said medicines comprise pharmaceutical compositions, which may contain optional pharmaceutical carrier.

In the present invention, said polypeptide and related peptidomimetic, including the functional fragment or variant thereof, as an effective inhibitory factor of Anti-FAS, is capable of inhibiting the transcription or expression of FAS, and can therefore prevent and treat the occurrence and development of tumors, fatty liver and obesity, including inhibition of the growth and development of liver cancer. It is worth of emphasizing that above-described polypeptides provided by the present invention are water soluble, stable, and of no immunogenic, and have significant pharmacodynamic effects, and therefore can be an effective medicine for the treatment of the above diseases.

<u>Brief Description of Drawings</u>

Figure 1. Analysis result of purified artificially synthesized polypeptide Anti-FAS-P18 by High Pressure Liquid Chromatography (HPLC) that shows the purity of obtained polypeptides is 98.11%.

Figure 2. Analysis of the effects of the plasmids containing the invention polypeptide genes on the SREBP-1c and FAS promoters in hepatoma cells by reporter gene assay. Results show that plasmid p-Anti-FAS-P18 inhibits the activities of the SREBP-1c and FAS promoters in HepG2 cells in a dose-dependent manner, which indicates the expressed product has a function of inhibiting the transcription and expression of FAS gene. Student's *t*-test is employed for statistical analysis, * $P<0.05$, ** $P<0.01$.

Figure 3. Analysis of the effects of the invention polypeptides and variants thereof on the activities of SREBP-1c and FAS promoters in the HepG2 cells. Results show that artificially synthesized polypeptide p-Anti-FAS-P18 inhibits the activities of the SREBP-1c and FAS promoters in the HepG2 cells in a dose-dependent manner, which indicates the invention polypeptides are capable of inhibiting the transcription and expression of FAS gene. In addition, 100 μM variants of the invention polypeptides p-Anti-FAS-P18, including P18-1 to P18-5, also inhibit the activities of the SREBP-1c and FAS promoters to different extent. Student's *t*-test is employed for statistical analysis, * $P<0.05$, ** $P<0.01$.

Figure 4. Analysis of the effects of the plasmids containing invention polypeptide gene on the expression level of FAS protein in hepatoma cells by immunoblotting. Results show that artificially synthesized p-Anti-FAS-P18 inhibits the expression level of FAS protein in HepG2 cells in a dose-dependent manner.

Figure 5. Analysis of the effects of the invention polypeptide on the expression level of FAS protein in the hepatoma cells by immunoblotting. Results show that artificially synthesized polypeptide p-Anti-FAS-P18 inhibits the expression level of FAS protein in HepG2 cells in a dose-dependent manner.

Figure 6. Analysis of the effects of the plasmids containing the invention polypeptide gene on activity of the NF-κB promoter in hepatoma cells by reporter gene assay. Results show that p-Anti-FAS-P18 inhibits the activity of NF-κB promoter in HepG2 cells in a dose-dependent manner, which indicates the decreasing of the capacity of the hepatoma cell in cell proliferation. Student's *t*-test is employed for statistical analysis, * $P<0.05$, ** $P<0.01$.

Figure 7. Analysis of the effects of the invention polypeptide and variants thereof on the activity of NF-κB promoter in hepatoma cells by reporter gene assay. Results show that p-Anti-FAS-P18 inhibits the activity of NF-κB promoter in HepG2 cells in a dose-dependent manner, which indicates that the capacity of the hepatoma cells in cell proliferation decreases. 100 μM variants of the invention polypeptides p-Anti-FAS-P18, including P18-1 to P18-5, also inhibit the activity of the NF-κB promoter to different extent. Student's t-test is employed for statistical analysis, * $P<0.05$, ** $P<0.01$.

Figure 8. Analysis of the effects of the plasmids containing the invention polypeptide genes on cell proliferation of the hepatoma cells by MTT assay. Results show that p-Anti-FAS-P18 inhibits cell proliferation of the HepG2 cells in a dose-dependent manner. Student's *t*-test is employed for statistical analysis, * $P<0.05$, ** $P<0.01$.

Figure 9. Analysis of the effects of the invention polypeptide and variants thereof on cell proliferation of the hepatoma cells by MTT assay. Results show that artificially synthesized polypeptide Anti-FAS-P18 inhibits cell proliferation in the HepG2 cells in a dose-dependent manner. 100 μM variants of the invention polypeptides p-Anti-FAS-P18, including P 18-1 to P18-5, also inhibit cell proliferation in the HepG2 cells to different extent. Student's t-test is employed for statistical analysis, * $P<0.05$, ** $P<0.01$.

Figure 10. The effect of synthesized polypeptide Anti-FAS-P18 on the growth of HepG2 cells. The results of inoculation experiment in nude mice show that Anti-FAS-P18 inhibits the growth and proliferation of the HepG2 cells. Student's t-test is employed for statistical analysis, ** $P<0.01$.

Figure 11. The effect of synthesized polypeptide Anti-FAS-P18 (P18) on the weight of nude mice. The results of inoculation experiment in nude mice show that Anti-FAS-P 18 has no significantly impact on the weight of nude mice.

Figure 12. Schematic diagram of the construction of the eukaryotic expression vector containing the gene encoding the invention polypeptide.

Detailed Description

In the present invention, including the description and claims, unless otherwise specified, the following terms are used with the following meanings:

"Isolated" refers to separating a substance from its original environment (e.g., its natural environment if it is naturally generated). For example, a naturally generated polynucleotide or polypeptide existing in a live animal means it has not been separated, whereas the same polynucleotide or polypeptide separated partially or completely from the natural systems with which it usually coexist means it is separated. Such a polynucleotide or polypeptide may exist as a part of a vector, or a part of a composition; since the vector and the composition are not a component of its natural environment, they are still separated.

The term "purified" as used herein means an increased status in purity , wherein "purity" is a relative term, and should not be narrowly construed as absolute purity. For example, the purity can be at least above about 50% , or greater than 60% , or than 70%, or than 80% , or than 90%, or even reach to 100%.

As used in the present invention, the isolated substance is separated from its original environment. The polynucleotides and polypeptides existed within the living cells are not isolated; however, the same polynucleotides and polypeptides that are separated from the substances that they usually coexist in the natural state should be regarded as be separated, while the purity is improved, and thus is purified.

The term "nucleic acid sequence", "nucleotide sequence" or "base pair sequence" used herein refer to nucleotide, oligonucleotide, polynucleotide and fragments or portions thereof. They generally refers to DNA (e.g., cDNA or genomic DNA) or RNA (e.g., mRNA), which can be single-stranded or double-stranded. Single-stranded DNA or RNA

can be coding strand (sense strand) or noncoding strand (antisense strand). Additionally, a polynucleotide referred in the present invention can also at its 5' terminal or 3' terminal be fused with tag (tag sequence or marker sequence). Synthesized or obtained (e.g., isolated and/or purified) from natural sources, they can contain natural, non-natural or altered nucleotides, and can contain natural, non-natural or altered internucleotide bond, such as a phosphoroamidate bond or a phosphorothioate bond , instead of the phosphodiester bond found between the nucleotides of an unmodified oligonucleotide.

The so-called "amino acid sequence" or "polypeptide" refers to a peptide, oligopeptide, polypeptide or protein and their partial fragment , which consists of amino acids that are connected with each other by peptide bonds. When amino acid sequence in the present invention is related to the amino acid sequence of a naturally occurred protein molecule, "polypeptide" or "protein" is not meant to limit the amino acid sequence for the entire natural amino acid sequence of said protein molecules. The amino acid sequence of the present invention may contain additional peptides. Labeled peptide epitope can be additional peptides, such as multiple histidine tag (His-tag), or myc, flag, etc.

"Deletion" refers to the deletion of one or a plurality of amino acids or nucleotides from the amino acid sequence or nucleotide sequence, respectively.

"Insert" or "add" refers to the increasing of one or a plurality of amino acids or nucleotides caused by the change of amino acid sequence or nucleotide sequence, respectively, compared with the molecules of the natural presence or before the change.

"Substitution" refers to one or a plurality of amino acids or nucleotides are replaced by different amino acids or nucleotides.

"Deletion, substitution or addition of one or plural amino acids or nucleotides" refers to the use of known methods of mutating nucleic acid or polypeptide such as directed mutagenesis method to delete, substitute or add one or a plurality of the number of amino acid or nucleoside acid, or a naturally occurring nucleic acid or polypeptide of the mutation being separated and purified. Mutation of amino acids can contain one or a plurality of amino acid residues with the conformation of D-amino acids, rare amino acids, naturally occurred or even artificially modified amino acids, and these amino acids may or may not be encoded by the genetic codon. Similarly, the induction of nucleic acid mutation can include the naturally occurring nucleotide, and may also include a modified nucleotide.

The "functional fragment" of a polypeptide as used herein refers to any part or portion of the polypeptide of the invention, which retains the substantially similar or identical biological activity of the polypeptide of which it is a part (the parent polypeptide). The "functional variant" of a polypeptide as used herein refers to amino acid sequences having substantial similar or identical biological activity of a polypeptide, including, for example, 1) the original amino acid sequence with one or more amino acid residues deletion and/or one or more amino acid residues addition; or 2) one or more of the amino acid residues in the original amino acid sequence substituted by one or more conserved or non-conserved amino acid residues; or 3) a group in one or more amino acid residues of the amino acid sequence substituted by other group; or 4) the original amino acid sequence fused with another molecule or compound (such as sugar, lipids, polyethylene glycol, etc.); or 5) original amino acid sequence fused with additional polypeptide sequences (e.g., leader sequence or a secretion signal sequence or polypeptide sequence used for purifying purpose); or 6) the retroinverso analogue of the original amino acid sequence; or 7) mixed of above. In the present invention, the functional variant of an amino acid sequence may contain one or more D-type amino acids, rare amino acids that are naturally occurred, or artificially modified amino acids, these amino acids may or may not be encoded by the genetic code.

The term "retroinverso analogue" refers to a polypeptide comprising a revered amino acid sequence of a parent polypeptide, such that the amino acid sequence of the retroinverso analogue (when read from the N-terminus to the C-terminus) is the same as the amino acid sequence of the parent polypeptide when read from the C-terminus to the N-terminus. Furthermore, with respect to a retroinverso analogue, each of the amino acid is the D isomer of the amino acid, as opposed to the L isomer. For example, the retroinverso analogue of the tripeptide Val-Ala-Gly has an amino acid sequence Gly-Ala-Val, and each of the amino acid is the D isomer.

In the present invention, the term "peptidomimetic" refers to a compound which has essentially the same general structure of a corresponding polypeptide with modifications that increase its stability or biological function. A peptidomimetics includes, for example, those compounds comprising the same amino acid sequence of a corresponding polypeptide with an altered backbone between two or more of the amino acids. The peptidomimetics can comprise synthetic or non-naturally occurred amino acids in place of naturally-occurred amino acids.

In the present invention, "degenerate variant" of the nucleotide sequence is such a polynucleotide sequence that is different from the parent nucleotide sequence, but encodes the same protein or polypeptide as the parent nucleotide sequence does.

"Nucleic Acid Hybridization" is known in the art (see, e.g., Sambrook et al, Molecular Cloning: A Laboratory Manual, 3rd Ed., Cold Spring Harbor Laboratory, 2001). In general, the higher the temperature is, the lower the salt concentration is, the more rigor the hybridization condition becomes (more difficult to hybridize), thereby obtaining more similar polynucleotides. The proper hybridization temperature varies depending on the length of the nucleotide sequence or its base-pair sequence. Further, the present invention also relates to hybridization under "stringent condition." The

term "stringent conditions" in the present invention refers to hybridization and elution in a condition of low ionic concentration and high temperature. For example, incubation overnight at 42°C, (50% formamide, 5 x SSC (150 mM NaCl, 15 mM citric acid tri-sodium), 50 mM sodium phosphate (pH 7.6), 5 X Denhardt's solution, 10% sulfuric acid dextran, and 20 μg/ml of denatured cut salmon sperm DNA), and then elution with 0.1 X SSC at 65°C.

"Homology" refers to the degree of complementation, which may be partially homologous, or may be completely homologous. "Partial homologous" refers to a partially complementary sequence, which can partially inhibit hybridization between the target nucleotide with a completely complementary sequence. This inhibition may be detected by hybridization under reduced rigor condition (southern blot or northern blot, etc.). Substantially homologous sequence or hybridization probe can compete and inhibit the binding of the completely complementary sequence to the target sequence under reduced rigor condition. Of course, reduced rigor condition does not allow non-specific binding. The two sequences combined with each other still require a specific interaction.

The percentage of "homology" or "identity" of amino acid sequence or nucleotide sequence refers to a percentage of sequence identity or similarity in the comparison with two or more amino acid sequences or nucleotide sequences. There are many methods to determine the percentage of sequence identity for the skilled artisan, such as the MEGALIGN program (Lasergene Software Packages, DNASTA Inc., Madison, WI). MEGALIGN program can compare two or more sequences based on the different types of method such as the Cluster Method (see Higgins & Sharp, Gene 73:237-244 (1988)). Each set of sequence is aligned by clusters by checking the distance between the pairs, and then the cluster is assigned by pairs or groups. The percentage of similarity of two amino acid sequences, sequence A and sequence B, can be calculated by the following formula:

[(the number of residues matching between Sequence A and Sequence B) / (the number of residues of sequence A - residues of the sequence A in the intervals - residues of sequence B in the intervals)] X 100%

Similarly, it can be calculated by Cluster method or methods known in the art, such as the Jotun Hein method (see Hein J., the Methods in Emzumology 183:625-645, 1990) to determine the percentage of the similarity between two nucleic acid sequence.

The term "recombinant expression vector" means a genetically-modified recombinant oligonucleotide or polynucleotide, which comprises nucleotide sequence encoding mRNA, protein, polypeptide, and peptide when the recombinant vector is contacted with the host cell under conditions sufficient to have the mRNA, protein, polypeptide or peptide expressed within the cell. The invention recombinant expression vector can comprise any type of nucleotides, including, but not limited to DNA and RNA, which can be single-stranded or double-stranded, synthesized or obtained in part from natural sources , and which can contain natural, non-natural or altered nucleotides. The linkage between nucleotide can be naturally-occurring, and can also be non-naturally-occurring or modified.

In the present invention, "treatment" and "prevention" and words derived from them, do not mean 100% or completely treatment or prevention, but can be identified as the treatment or prevention extent approved by those skilled. In the present invention, "prevention" could be understood as delay the onset of the disease, or its symptoms or disorders.

Polypeptide

The present invention relates to isolated or purified polypeptides. The polypeptides comprise, basically consist of, or consist of the amino acid sequence as follows: Gly-Gly-Cys-Arg-His-Lys-Leu-Val-Cys-Ala-Pro-Ala-Pro-Cys-Asn-Phe-Ph e-Thr (SEQ ID NO: 1).

The expression of FAS gene is regulated by its upstream regulatory factor, i.e., the steroid regulatory element binding protein 1c (SERBP-1c). When the transcription of SERBP-1c is inhibited, the expression level of FAS gene is down-regulated. Therefore, the regulation of the activity of the SERBP-1c promoter can indirectly regulate the expression of FAS gene. A polypeptide of the present invention can play the role of inhibiting the transcription and expression of FAS by inhibiting the transcription of SERBP-1c, thereby can be used for preventing and treating the occurrence and development of tumors, fatty liver and obesity, including inhibition the occurrence and development of hepatoma cells as well as liver cancer.

The inhibition is reflected at the molecular level, the cellular level and the *in vivo* level. For example, said polypeptides at the molecular level can inhibit the SREBP-1c and FAS promoter activity, and down-regulates the expression level of FAS protein, thereby effectively inhibiting FAS, and the inhibitory effects thereof are dose-dependent; said polypeptides also have inhibitory effective at the cellular level, such as effectively inhibiting the growth and proliferation of hepatoma cells, and the inhibitory effects thereof are also dose-dependent; nude mice inoculation experiments further show that the invention polypeptides can effectively inhibit the oncogenicity of tumor cells, whereas they show no effect on the weight of nude mice.

The present invention also provides various functional fragments of the polypeptide. Said functional fragments can be any fragments of the continuous amino acid sequence of the polypeptide of the present invention on condition that the functional fragments can retain the parent polypeptide's biological activity by a similar extent, by the same extent, or by a higher extent compared with the parental polypeptide, e.g., inhibiting HBx activity. With reference to the parent polypeptide, the functional fragments can have, e.g., approximately 10%, 20%, 30%, 40%, 50%, 60%, 70%, 80%, 90%, 95%, 100%, 105%, 110%, 120%, 150%, 200% or even greater activity of that provided by the parent polypeptide. It is preferred that the functional fragments of the polypeptide of the present invention include the amino acid sequence having at least 70% identity to said parent polypeptide.

In addition, the functional variants of the polypeptide and the functional fragments thereof in the present invention are also included within the scope of the invention. The functional variants of the polypeptide and functional fragments thereof in the present invention retains substantial similar or identical biological activity with the parent polypeptide or the functional fragments thereof, e.g., inhibition of the transcription and expression of FAS, and effectively inhibit cell growth and proliferation in tumor cells. With reference to parent polypeptide or the functional fragment thereof, the functional fragment can have, e.g., approximately 50%, 60%, 70%, 80%, 90%, 95% or 100% identity in the amino acid sequence. Preferably, the functional variants of the polypeptide and functional fragments thereof according to the present invention include the sequence having at least 70% sequence identity with that of the parent polypeptide or the functional fragment thereof. More preferably, the functional variant of the invention polypeptide and the functional fragments thereof differ from the parent polypeptide and the functional fragment thereof by only 1-3 amino acids. In some particularly preferred embodiments, the functional variant of the polypeptide and functional fragments thereof according to the present invention differ from the parent polypeptide and the functional fragment thereof by only one amino acid.

The polypeptides and the functional fragments or variants thereof can comprise the amino acid sequence of the parent polypeptide or parent functional fragment with conservative amino acid substitutions. The functional variant can, for example, comprise the amino acid sequence of the parent polypeptide or parent functional fragment with at least one conservative amino acid substitution. Specifically, the functional variant can comprise the amino acid sequence of the parent polypeptide or parent functional fragment with two, three, four, five, or more conservative amino acid substitutions. Alternatively, the functional variants can comprise the amino acid sequence of the parent polypeptide or parent functional fragment with at least one non-conservative amino acid substitution. Specifically, the functional variants can comprise the amino acid sequence of the parent polypeptide or the functional fragments thereof with two, three, four, five, or more non-conservative amino acid substitutions. In this case, it is preferable for the non-conservative amino acid substitutions to not interfere with or inhibit the biological activities of the functional variants. Preferably, the non-conservative amino acid substitution enhances the biological activity of the functional variant, such that the biological activity of the functional variant is increased as compared to the parent polypeptide or functional fragment thereof.

The polypeptides and the functional fragments in this invention and their functional variants preferably comprise one or more conservative amino acid substitutions. Conservation amino acids substitutions are known in the art, and include amino acid substitutions in which one amino acid having certain physical and/or chemical properties is exchanged for another amino acid that has the same chemical or physical properties. Skilled in the art understand that conservative amino acid substitutions can not cause significant changes in the structure or function of the protein. For instance, the conservative amino acid substitution can be an acidic amino acid substituted for another acidic amino acid (e.g., Asp or Glu), an amino acid with a nonpolar side chain substituted for another amino acid with a nonpolar side chain (e.g., Ala, Gly, Val, Ile, Leu, Met, Phe, Pro, Trp, Val, etc.), a basic amino acid substituted for another basic amino acid (Lys, Arg, etc.), an amino acid with a polar side chain substituted for another amino acid with a polar side chain (Asn, Cys, Gln, Ser, Thr, Tyr, etc.), an aromatic amino acid (Trp, Phe, Tyr, etc.) for another aromatic amino acid, etc.

Said functional variants can add and/or delete one or plural amino acids in the middle of said polypeptides and functional fragments thereof, and/or in the amino-terminus or carboxyl-terminus. Preferably, said addition or deletion of amino acids have no impact on the biological function and activity of said polypeptides and functional fragments thereof, for example, inhibition of the transcription and expression of FAS, and subsequently effectively inhibiting tumor cells, including the growth and proliferation of the tumor cells. More preferably, when comparing with the biological activity of said parent polypeptide, the sequence with extra or missed amino acid can have enhanced biological activity.

Alternatively, the functional variants may comprise a retroinverso analogue of any of the invention polypeptides or functional fragments thereof. The term "retroinverso analogue" refers to a polypeptide comprising a revered amino acid sequence of a parent polypeptide, such that the amino acid sequence of the retroinverso analogue (when read from the N-terminus to the C-terminus) is the same as the amino acid sequence of the parent polypeptide when read from the C-terminus to the N-terminus. Furthermore, with respect to the retroinverso analogue, each of the amino acid is the D isomer of the amino acid, as opposed to the L isomer. For example, the retroinverso analogue of the tri-peptide Val-Ala-Gly has an amino acid sequence Gly-Ala-Val, in which each amino acid is the D isomer. With respect to this

invention, the functional variants preferably comprise a retroinverso analogue of SEQ ID NO: 1.

The polypeptides,the functional fragments thereof, and their functional variants according to the invention can be of any length, i.e., can comprise any number of amino acids , provided that the polypeptide and the functional fragments thereof and their functional variants retain the essential biological activities of the parent polypeptide, e.g., the ability to inhibit the transcription and expression of FASactivity of, and effectively inhibit cancer cells, preferably liver cancer cells. For example, the invention polypeptide or the peptidomimetic can be 4 to 2000 amino acids long, such as 5 , 6, 7 , 8, 9 , 10 , 11, 12, 13, 14 , 15, 16, 20, 25, 30, 40, 50 , 70, 75, 100, 125 , 150, 175, 200, 300, 400, 500, 600, 700 , 800, 900, 1000 or more amino acids in length. Preferably, the polypeptides of the invention have less than 30 amino acids in length, and meet the requirements of the pharmacodynamics and half-life of polypeptide drug.

In some particular embodiment, the functional variants of the parent polypeptide and the functional fragments thereof according to the present invention have one or plural amino acids different from the parent polypeptide of SEQ ID NO: 1, e.g. including one, two ,three, four, five amino acids that are in difference. These amino acid sequence can be obtained by adding or deleting one or plural amino acid residues in the middle or terminal of SEQ ID NO:1, or substituting one or plural amino acid residues in the SEQ ID NO:1. The above-obtained functional variants have very similar biological activity and function with the parent polypeptide of SEQ ID NO: 1, for example, inhibition of the transcription and expression of FAS, and effectively inhibiting cancer cells, preferably liver cancer cells. More preferably, these amino acid sequence can be obtained by adding or deleting one amino acid residues in the middle or terminal of SEQ ID NO:1, or substituting one amino acid residues in the SEQ ID NO:1. In a particular embodiment, the invention polypeptide in the present invention include any amino acid sequence of SEQ ID NOs: 1-6.

Also provided by the invention are peptidomimetics of any of the invention polypeptides (including functional fragments and functional variants) described herein. In a preferred embodiment, the peptidomimetics is a peptoid. The term "peptoid" as used herein refers to a peptidomimetics in which the side chains of each amino acid are appended to the nitrogen atom of the amino acid as opposed to the alpha carbon. For example, peptoids can be considered as N-substituted glycines which have repeating units of the general structure of $NRCH_2CO$ and which have the same or substantially the same amino acid sequence as the corresponding polypeptide. In another preferred embodiment, the peptidomimetics comprises an altered backbone in which the bond between each amino acid is methyalted. In this regard, the peptidomimetics can comprise a methylated peptide backbone of the following structure:

$$\ldots NCH_3 - C_\alpha - CO - NCH_3 - C_\alpha - CO \ldots$$

Side Chain Side Chain

The polypeptide (including functional fragments and functional variants) and peptidomimetics of the invention can comprise synthetic amino acid in place of one or more naturally-occurred amino acids. Such synthetic amino acids are known in the art, and include, for example, aminocyclohexane carboxylic acid, norleucine, α-amino n-decanoic acid, homoserine, S-acetylaminomethyl-cysteine, trans-3-hydroxyproline, trans-4-hydroxyproline, 4-aminophenylalanine, 4-benzoylphenylalanine, 4-nitropheylalanine, 4-chlorophenylalanine, 4-carboxyphenylalanine, β-phenylserine, β-hydroxyphenylalanine, phenylglycine, α-naphthylalanine, cyclohexylalanine, cyclohexylglycine, indoline-2-carboxylic acid, 1,2,3,4-tetrahydroisoquinoline-3-carboxylic acid, aminomalonic acid, aminomaloinc acid monoamide, N'-benzyl-N'-methyl-lysine, N',N'-dibenzyl-lysine, 6-hydroxylysine, ornithine, α-aminocyclopentane carboxylic acid, α-aminocyclohexane carboxylic acid, α-aminocycloheptane carboxylic acid, α-(2-amino-2-norbornane)-carboxylic acid, α,γ-diaminobutyric acid, α,β-diaminopropionic acid, homophenylalanine, and α-tert-butylglycine.

The polypeptides (including functional fragments and functional variants) and peptidomimetics of the invention can be lipidated (e.g., fatty acidated), glycosylated, amidated, carboxylated, phosphorylated, esterified, N-acylated, cyclized via, e.g., a disulfide bridge, or converted into an acid addition salt and/or optinally dimerized or polymerized, and/or conjugated.

For instance, the polypeptides (including functional fragments and functional variants) and peptidomimetics can be lipidated derivatives. The lipid can be any lipid known in the art, such as, for example, a fatty acid, a farnesyl group (e.g., farnesyl diphosphate), a geranylgeranyl group (e.g., geranylgeranyl diphosphate), a phospholipid group, glycophosphatidylinositol, phosphatidylserine, phosphatidylethanolamine, sphingomyelin, phoshpatidylcholine, cardiolipin, phosphatidylinositol, phosphatidic acid, lysophosphoglyceride, and a cholesterol group. Preferably, the lipidated derivative is a fatty acid derivative in which the polypeptide or peptidomimetics described herein comprises a fatty acid molecule. The fatty acid molecule can be any C8-C20 fatty acid. The fatty acid molecule can be, e.g., lauric acid, palmitic acid, myristic acid, stearic acid, oleic acid, linoleic acid, linolenic acid, arachidonic acid, timnodoinc acid, erucic acid, or arachidic acid. The fatty acid may optionally contain additional functional groups, e.g., one or more amino groups on

any of the carbon atoms. The fatty acid molecule can be attached to any suitable part of the inventive polypeptide (including functional fragment and functional variant) or peptidomimetics. For instance, the inventive polypeptide comprises a fatty acid molecule at the amino terminus, the carboxyl terminus, or both the amino and carboxyl termini. The fatty acid molecule can be attached to the inventive polypeptide (including functional fragment and functional variant) or peptidomimetics directly or through a linker.

The polypeptides (including functional fragments and functional variants) and peptidomimetics according to the invention, including fatty acid derivatives thereof, can be a monomer peptide, or can be a dimmer or multimer peptide.

The polypeptides of the invention (including functional fragments and functional variants) and peptidomimetics can be obtained by methods known in the art (See, for instance, Chan et al., Fmoc Solid Phase Peptide Synthesis, Oxford University Press, Oxford, United Kingdom, 2005 ; Reid, R., Peptide and Protein Drug Analysis, Marcel Dekker Company, 2000; and U.S. Patent No. 5,449,752). Also polypeptides can be recombinantly produced using the nucleic acids described herein using standard recombinant methods (See, for instance, Sambrook et al., Molecular Cloning: A Laboratory Manual 3rd ed., Cold Spring Harbor Press, Cold Spring Harbor, NY 2001). Further, some of the polypeptides of the invention (including functional fragments and functional variants thereof) can be isolated and/or purified from a source, such as a plant, a bacterium, an insect, a mammal, e.g., a rat, a human, etc. Methods of isolation and purification are well-known in the art. Alternatively, the polypeptides described herein (including functional fragments and functional variants thereof) can be synthesized or obtained from commercial companies.

Polynucleotide

The present invention also provides isolated polynucleotides encoding any one of said polypeptide of the present invention (including the functional fragments and functional variants thereof). Said polynucleotide comprises the polypeptide (including functional fragments and functional variants thereof) coding sequence that encodes any one of the present invention polypeptides, e.g., the nucleotide sequence of SEQ ID NO: 7 encoding SEQ ID NO: 1, and any degenerate variant of the coding sequence; alternatively, the polynucleotide can also include additional coding sequence and/or non-coding sequence. In another aspect, the present invention also provides isolated polynucleotides or fragments that contains a nucleotide sequence complementary to the nucleotide sequence of any one of the nucleic acid in the present invention or hybridizes with the nucleotide sequence of any one of polynucleotide under stringent condition.

The present invention also provides variants of the polynucleotide, encoding same amino acid sequence of the polypeptides or polypeptide fragment, functional variant with the polypeptide (including functional fragments and functional variant) in the present invention. These polynucleotide variants can be naturally occurring allelic variants or non-naturally occurring variants. These nucleotide variants include substituted variants, deletion variants, and insertion variants. As known in the art, allelic variant is an alternate form of a polynucleotide, it may contain one or more nucleotide substitutions, deletions or insertions, but will not substantially alter the function of the encoded polypeptide.

The nucleic acids can be purified from natural-occurring, and also can be produced by recombination, or can be constructed based on chemical synthesis and/or enzymatic ligation reactions using procedures known in the art. For example, a nucleic acid can comprise naturally occurring nucleotides or variously modified nucleotides designed to increase the biological stability of the molecules or to increase the physical stability of the duplex formed upon hybridization (e.g., phosphorothioate derivatives and acridine substituted nucleotides). Examples of modified nucleotides that can be used to generate the nucleic acids include, but are not limited to, 5-fluorouracil, 5-bromouracil, 5-chlorouracil, 5-iodouracil, hypoxanthine, xanthine , 4-acetylcytosine , 5-(carboxyhydroxymethyl) uracil, 5-methoxyaminomethyl-2-thiouracil, uracil-5-oxyacetic acid, 5-methyl-2-thiouracil, 2-thiocytosine, 2-thiouracil, 4-thiouracil, 5-methyluracil, uracil-5-oxyacetic acid methylester, 3-(3-amino-3-N-2-carboxypropyl) uracil, and 2,6-diaminopurine and the like. Further, it can contain natural or altered linkages other than the phosphodiester bond between nucleotides, such as phosphoramidate linkages. Preferably, nucleic acids used in this invention are produced through recombination.

Recombinant Expression Vector

The invention further provides any recombinant expression vector containing the inventive polynucleotide. The recombinant expression vector of the invention can be any suitable recombinant expression vector, and can be used to transform or transfect any suitable host. Suitable vectors include those designed for propagation and expansion or for expression or both , such as plasmids and viruses. The vector can be selected from the group consisting of the pUC series, the pcDNA series, the pBluescript series, the pET series, the pGEX series, and the pEX series. Bacteriophage vectors, such as λGT10, λGT111, λZapII, λEMBL4, etc. also can be used. Examples of plant expression vectors include pBI01, pBI101.2, pBI101.3, pBI121 and pBIN19. Examples of animal expression vectors include pEUK-C1, pMAM and pMAMneo. Preferably, the recombinant expression vector is pcDNA series.

The recombinant expression vectors of the invention can be prepared using standard recombinant DNA techniques. Constructs of expression vectors, which are circular or linear, can be prepared to contain a replication system

functional in a prokaryotic or eukaryotic host cell. Desirably, the recombinant expression vector comprises regulatory sequences, such as transcription and translation initiation and termination codons, which are specific to the type of host (e.g. , bacterium, fungus, plant , or animal) into which the vector is to be introduced, as appropriate and taking into consideration whether the vector is DNA- or RNA- based.

The recombinant expression vector can include one or more marker genes, which allow for selection of transformed or transfected hosts. Marker genes include biocide resistance, e.g., resistance to antibiotics, heavy metals, etc., complementation in an auxotrophic host to provide prototrophy, and the like. Suitable marker genes for the inventive expression vectors include , for instance, neomycin/G418 resistance genes, hygromycin resistance genes , histidinol resistance genes , tetracycline resistance genes, and ampicillin resistance genes.

The recombinant expression vector can comprise a native or normative promoter. The selection of promoters , e.g. , strong, weak, inducible, tissue-specific and developmental-specific, is within the ordinary skill of the artisan. Similarly, the combining of a nucleotide sequence with a promoter is also within the skill of the artisan. The promoter can be a non-viral promoter or a viral promoter , e.g., a cytomegalovirus (CMV) promoter, an SV40 promoter , an RSV promoter , and a promoter found in the long-terminal repeat of the murine stem cell virus. The inventive recombinant expression vectors can be designed for either transient expression, for stable expression , or for both. Also, the recombinant expression vectors can be made for constitutive expression or for inducible expression.

Further , the recombinant expression vectors can be made to include a suicide gene. The term "suicide gene" refers to a gene that causes the cell expressing the suicide gene to die. The suicide gene can be a gene that confers sensitivity to an agent, e.g., a drug, upon the cell in which the gene is expressed , and causes the cell to die. Suicide genes are known in the art (see, for example, Suicide Gene Therapy: Methods and Reviews, Springer, Caroline J. (Cancer Research UK Centre for Cancer Therapeutics at the Institute of Cancer Research, Sutton, Surrey, UK), Humana Press , 2004) and include, for example, the Herpes Simplex Virus (HSV) thymidine kinase (TK) gene , cytosine daminase , purine nucleoside phosphorylase, and nitroreductase.

## Host Cell

The invention farther provides a host cell comprising any of the recombinant expression vectors described herein. As used herein, the term "host cell" refers to any type of cell that can contain the inventive recombinant expression vector. The host cell can be a eukaryotic cell, e.g. , plant, animal, fungi, or algae, or can be a prokaryotic cell, e.g., bacteria or protozoa. The host cell can be a cultured cell or a primary cell, i.e. , isolated directly from an organism, e.g., a human. The host cell can be an adherent cell or a suspended cell, i.e. , a cell that grows in suspension. Suitable host cells are known in the art and include, for instance, DH5a E. coli cells, Chinese hamster ovarian cells , monkey VERO cells , COS cells, HEK293 cells, and the like. For purposes of amplifying or replicating the recombinant expression vector, the host cell is preferably a prokaryotic cell , e.g., a DH5$\alpha$ cell. For purposes of producing a recombinant modified TCR, polypeptide, or protein, the host cell is preferably a mammalian cell. Most preferably, the host cell is a human cell. The host cell can be of any cell type , can originate from any type of tissue , and can be of any developmental stage.

Also provided by the invention is a population of cells comprising at least one host cell described herein. The population of cells can be a heterogeneous population comprising the host cell comprising any of the recombinant expression vectors described, in addition to at least one other cell , e.g., a host cell (e.g., a T cell), which does not comprise any of the recombinant expression vectors, or a cell other than a T cell, e.g., a B cell , a macrophage, a neutrophil, an erythrocyte, a hepatocyte, an endothelial cell, an epithelial cells, a muscle cell, a brain cell, etc. Alternatively, the population of cells can be a substantially homogeneous population, in which the population comprises mainly of host cells (e.g., consisting essentially of) comprising the recombinant expression vector. The population also can be a clonal population of cells, in which all cells of the population are clones of a single host cell comprising a recombinant expression vector, such that all cells of the population comprise the recombinant expression vector. In one embodiment of the invention, the population of cells is a clonal population comprising host cells comprising a recombinant expression vector as described herein.

## Conjugate

Included in the scope of the invention are conjugates, e.g., bioconjugates, comprising any of the inventive polypeptides (including any of the functional fragments or functional variants) or peptidomimetics , nucleic acids, recombinant expression vectors , or host cells. Conjugates, as well as methods of synthesizing conjugates in general, are known in the art (See, for instance, Hudecz, F., Methods Mol Biol. 298: 209-223 (2005) and Kirin et al., Inorg Chem. 44(15): 5405-5415 (2005)).

Pharmaceutical Composition

The inventive polypeptides (including functional fragments and functional variants), peptidomimetics, fatty acid derivatives, conjugates, nucleic acids , recombinant expression vectors, and host cells (including populations thereof), all of which are collectively referred to as "inventive materials" hereinafter, can be isolated, purified, synthesized and/or recombinated.

The inventive materials also can be formulated into a composition, such as a pharmaceutical composition. In this regard , the invention provides a pharmaceutical composition comprising any of the polypeptides (including functional fragments and functional variants), peptidomimetics, fatty acid derivatives, conjugates, nucleic acids , recombinant expression vectors, and host cells (including populations thereof), and a pharmaceutically acceptable carrier. The inventive pharmaceutical compositions containing any of the inventive materials can comprise more than one inventive material, e.g., a polypeptide and a nucleic acid, or two or more different polypeptides. Alternatively , the pharmaceutical composition can comprise an inventive material in combination with another pharmaceutically active agent or drag , such as a chemotherapeutic agent , e.g., asparaginase, busulfan, carboplatin, cisplatin, damiorabicin, doxorubicin, fluorouracil, gemcitabine, hydroxyurea, methotrexate, paclitaxel , rituximab, vinblastine, vincristine, etc..

With respect to pharmaceutical compositions, the pharmaceutically acceptable carrier can be any of those conventionally used and is limited only by chemico-physical considerations, such as solubility and lack of reactivity with the active compound(s), and by the route of administration. The pharmaceutically acceptable carriers described herein, for example, vehicles, adjuvants, excipients , and diluents , are well-known to those skilled in the art and are readily available to the public. It is preferred that the pharmaceutically acceptable carrier be one which is chemically inert to the active agent(s) and one which has no detrimental side effects or toxicity under the conditions of use.

For purposes of the invention, the amount or dose of the inventive material administered should be sufficient to effect, e.g., a therapeutic or prophylactic response, in the subject or animal over a reasonable time frame. For example, the dose of the inventive material should be sufficient to inhibit proliferation of a diseased cell, or treat or prevent a disease (e.g., cancer, neoplasm, or psoriasis in a period of from about 2 hours or longer, e.g., 12 to 24 or more hours, from the time of administration. In certain embodiments, the time period could be even longer. The dose will be determined by the efficacy of the particular inventive material and the condition of the animal (e.g., human), as well as the body weight of the animal (e.g., human) to be treated. Many assays for determining an administered dose are known in the art. The dose of the inventive material also will be determined by the existence, nature and extent of any adverse side effects that might accompany the administration of a particular inventive material.

One of ordinary skill in the art will readily appreciate that the inventive materials of the invention can be modified in any number of ways , such that the therapeutic or prophylactic efficacy of the inventive materials is increased through the modification. For instance, the inventive materials can be conjugated either directly or indirectly through a linker to a targeting moiety. The practice of conjugating compounds , e.g., inventive materials, to targeting moieties is known in the art. See, for instance, Wadwa et al., J. Drug Targeting 3: 111 (1995) and U.S. Patent No. 5,087,616. In another embodiment, the inventive materials can be modified into a depot form, such that the manner in which the inventive materials is released into the body to which it is administered is controlled with respect to time and location within the body (see , for example, U.S. Patent No. 4,450,150). Depot forms of inventive materials can be, for example, an implantable composition comprising the inventive materials and a porous or non-porous material, such as a polymer, wherein the inventive materials is encapsulated by or diffused throughout the material and/or degradation of the non-porous material. The depot is then implanted into the desired location within the body and the inventive materials are released from the implant at a predetermined rate.

One of ordinary skill in the art will readily appreciate that the invention provides a method of preventing and inhibiting proliferation of a diseased cell. The method comprises contacting the diseased cell with any of the pharmaceutical compositions described herein in an amount effective to inhibit proliferation of the diseased cell. In a preferred embodiment of the inventive methods, the pharmaceutical composition is topically administered to the host. In another preferred embodiment, the pharmaceutical composition is administered directly to the tumor, e.g., delivered intratumorally.

The pharmaceutical compositions according to the invention, including polypeptides (including functional fragments and functional variants) and the peptidomimetics , nucleic acids , recombinant expression vectors, and/or host cells can be used in methods of preventing and treating tumors, fatty liver and obesity. Ordinary skill in the art should be readily understood that the diseases according to the present invention may be present in any host. Preferably, the host is a mammal. An especially preferred mammal is the human.

The present invention will be further illustrated below with reference to the specific examples. It should be understood that these examples are only used to describe the invention but not to limit the scope of the invention. The experimental methods with no specific conditions described in the following examples are generally performed under conventional conditions, and the materials used without specific description are purchased from common chemical reagents corporation.

Examples

Example 1: Design and preparation of polypeptides

Artificial synthesis of the fragment of polypeptides anti-FAS-P18:

The polypeptides having the amino acid sequence Gly-Gly-Cys-Arg-His-Lys-Leu-Val-Cys-Ala-Pro-Ala-Pro-Cys-Asn-Phe-Ph e-Thr (SEQ ID NO: 1) (hereafter called Anti-FAS-P 18) are synthesized by artificial synthetic methods. The polypeptide was prepared through solid phase peptide synthesis method, and was carried out on the Apex396 Peptide Synthesizer produced by AAPPTEC Company; the synthesis was performed in accordance with the sequence of SEQ ID NO: 1, from C-terminus carboxyl terminal to N-terminus amino terminal, to synthesize the amino acid in the sealed explosion-proof glass reactor. This refers to that the first amino acid monomer added into the amino acid sequence of Gly-Gly-Cys-Arg-His-Lys-Leu-Val-Cys-Ala-Pro-Ala-Pro-Cys-Asn-Phe-Ph e-Thr was Thr in the C-terminus, followed by Phe, and then Phe, until the last Gly was added in the N-terminus. The resulting peptide was obtained by repeating adding, reacting and synthesizing. The solid phase peptide synthesis was performed with conditional blocking of unreacted amino groups with acetic anhydride and activating unbounded carboxyl groups before reacting for easier purification of the resulting peptides.

The detailed synthesis cycles were shown as follow:

1) Remove of the protection: Removed the Fmoc protecting groups by treating the resin with a solution of basic solvent (piperidine);
2) Activation and coupling: The carboxyl group of the next amino acid is activated by activator. The activated monomer couples with unbounded amino group to form peptide bond. An amount of ultra concentration reagents were used in this step to drive the reaction complete. Cycle: repeat this two step reaction until the desired sequence of amino acids was obtained;
3) Wash and cleavage: The protecting groups of peptide were removed from the polyamide by cleaving the polyamide - peptide bond with protectant (TFA). The synthesis was performed from C-terminus (carboxyl terminal) to N-terminus (amino terminal). Fixed the first amino acid Thr in the C-terminus on the resin, and removed the protecting group of Thr, and then activated the carboxyl terminal of next amino acid Phe and so on, until the last amino acid was synthesized. The resulting peptide was cleaved from the resin, and purified by HPLC to obtain the 98% pure Anti-FAS-P18. Finally, the mass spectrum shows the molecular weight of the peptide is 1921.6Kd.

In the solid phase peptide synthesis, the elongation of peptide chain is performed on the polystyrene resin carrier. The C-terminus of synthesized peptide reacted with chloromethylated polystyrene (chlorinated benzyl ester resin) to form benzyl ester, and then added the amino acid with the protected amino group one by one in accordance with the primary structure of peptide chain to elongate the peptide chain.
The artificially synthesized polypeptide anti-FAS-P18 was analyzed by High Pressure Liquid chromatography (HPLC) (apply PLC Agela C 18 column), and shown the purity is 98.11%.

Example 2: The anti-FAS activities of polypeptides (*in vitro*)

Two methods were used to test the anti-FAS activities of the polypeptides in the Example 1 in vitro. One was that the cDNA expressed the polypeptides in the Example 1 was constructed into the eukaryotic expression vector pcDNA3.1(+) by molecular cloning technique, and then transfected into hepatoma cells to study the peptides, so as to observe the effects of studied polypeptides on inhibiting the FAS. The other one was that directly adding the synthesized polypeptides into the medium of cultured hepatoma cells, and then observe the effects of polypeptides on inhibiting the FAS.
Hepatoma cell line HepG2 was adopted in the experiment. Because SREBP-1c is upstream transcription regulatory factor of FAS, whether there is inhibition of SREBP-1c promoter activity at the molecular level can be used to reflect the transcription state of FAS gene by reporter gene assays. Meanwhile, the detection of FAS promoter activity can directly reflect whether the transcription of FAS is inhibited or not. The nuclear factor κB (nuclear factor κB, NF-κB) is an import transcription factor, and increasing of NF-κB promoter activity indicates the increase of cell proliferation. Therefore, it can test the effects of the invention polypeptides on the proliferation of HepG2 cell by detection of NF-κB promoter activity and (3-(4,5-dimethylthiazol-2-yl)-2,5-diphenyltetrazolium bromide (MTT).
A. The construction of polypeptides eukaryotic expression vector
1. Major materials :

1) Bacterial strain: *E. coli* DH5α (purchased from Yuanhaoping (Tianjin) Biological Technology Co., Ltd),
2) Plasmids: pcDNA3.1 (+) (purchased from Invitrogen), pEGFP-C2 purchased from Invitrogen).

2. Major reagents

| Name | Source |
|---|---|
| Agar | Solarbio, Beijing, China |
| Plasmid Extraction mini kit | Transgen, Beijing, China |
| Ampicillin | BBI, Boston Biotechnology, Inc.,USA |
| Trypsin | BBI, Boston Biotechnology, Inc.,USA |
| Tryptone | Promega, USA |
| Yeast extract | Promega, USA |
| EcoRI restriction enzyme | Takara, Dalian, China |
| XhoI restriction enzyme | Takara, Dalian, China |
| rTaq enzyme | Takara, Dalian, China |
| T4 DNA ligase | Takara, Dalian, China |
| Chloroform | BBI, Boston Biotechnology, Inc.,USA |

3. Major solution preparation :
1) LB solution

| | |
|---|---|
| Tryptone | 2.0g |
| Yeast extract | 1.0g |
| NaCl | 2.0g |
| Add dd$H_2O$ settled to | 200ml |

1.03×$10^5$ Pa, autoclave 20min.
2) LB solid medium

| | |
|---|---|
| Tryptone | 2.0g |
| Yeast extract | 1.0g |
| NaCl | 2.0g |
| Agar | 3.0g |
| Add dd$H_2O$ settled to | 200ml |

1.03×$10^5$ Pa, autoclave 20min.
3) 10mg/ml Ethidium bromide (EB)

| | |
|---|---|
| EB | 0.2g |
| dd$H_2O$ | 20ml |

Working solution: 0.5ug/ml
4) TBE electrophoretic buffer 5×Stock buffer:

| | |
|---|---|
| Tris-base | 54 g |
| Boric acid | 27.5 g |
| 0.5 mol/L EDTA (pH 8.0) | 20 ml |
| Add dd$H_2O$ settled to | 1000 ml |

4. Annealing system

| Reagents | Addition (μl) |
|---|---|
| $ddH_2O$ | 20 |
| 5 × Annealing Buffer | 10 |
| Forward DNA oligo (50 μM) (5'-AATTCATGGGAGGCTGTAGGCATAAATTGGTCTGCGCACCAGCACCATGCAACTTTTCACCTGAC-3') (SEQ ID NO:13) | 10 |
| Reverse DNA oligo (50 μM) (5'-TCGAGTCAGGTGAAAAGTTGCATGGTGCTGGTGCGCAGACCAATTTATGCCTACAGCCTCCCATG-3') (SEQ ID NO:14) | 10 |

5. Annealing conditions : 95°C, 2 min; decrease to 25°C at a rate of 0.1°C per 8 sec, 90min; 4°C, ∞.

6. pcDNA3.1 (+) mini-preparation of empty plasmid vector

Stored DH5α strain with pcDNA3.1 (+) plasmid was activated. Single colony was picked to add into LB medium (100mg/L ampicillin contained), and then cultured at 37°C overnight. TransGen Plasmid Extraction mini kit was used to extract the plasmids.

7. Double digestion reaction system:

| Reagent | Addition (μl) |
|---|---|
| Restriction enzyme EcoRI | 2 |
| Restriction enzyme XhoI | 2 |
| 10 × M buffer | 4 |
| DNA | Less than 2 μg |
| ddH2O | Settled to 40 μl |

After mixed, react at 37°Cfor 3-6 hr.

8. Ligation reaction system:

| Reagent | Addition (μl) |
|---|---|
| Annealing products | 0.3 pmol |
| Plasmid double digestion extracting products | 0.03 pmol |
| 10 × ligation buffer | 2.5 μl |
| Ligase | 1 μl |
| ddH2O | Setteled to 25 μl |

After mixed, react at 16°C overnight. Synthesized base pair sequence is ggaggctgta ggcataaatt ggtctgcgca ccagcaccat gcaacttttt cacc (SEQ ID NO:7), and restriction map as shown in the Figure 8.

9. Transformation step

1) Thaw DH5α competent cells (100 μl) on ice;
2) Put all the 25 μl ligation products in the Step 8 into 1.5ml tubes that have the competent cells, and gently mixed cells with the pipet tip. Incubated the tubes on ice for 30 min;
3) Heat shock at 42°C for exactly 90 sec;
4) Placed tubes on ice for 2 min immediately;
5) Added 500 μl LB without containing antibiotics, and then pre-culture at 37°C for 45 min;
6) Spread 100 μl bacteria solutions onto LB plates with 100mg/L ampicillin (appropriate adjust the amount according to the intensity of colonies), then allowed plates to dry and incubate inverted at 37°C overnight;

7) After single clear colonies grew on the plates, picked up a single colony to add into LB medium, and then cultured at 37°C overnight.

10. Colony PCR reaction system

| Reagents | Addition(μl) |
|---|---|
| $ddH_2O$ | 35.75 |
| 10 × buffer | 5 |
| dNTP (10mmol/L) | 4 |
| Forward Primer (20 μM) | 1 |
| 5'-TAATACGACTCACTATAGGG-3' (SEQ ID NO: 15) | |
| Reverse Primer (20 μM) | 1 |
| 5'-TAGAAGGCACAGTCGAGG-3' (SEQ ID NO:16) | |
| Template DNA (bacteria solution) | 3 |
| Taq enzyme | 0.25 |

11. Colony PCR reaction conditions:

| | | |
|---|---|---|
| 94°C | 10 min | |
| 94°C | 30 sec | 35 cycles |
| 53°C | 30 sec | |
| 72°C | 15 sec | |
| 72°C | 10 min | |
| 4°C | ∞ | |

After reaction, 1.5% agarose gel electrophoresis was undertaken to analyze the PCR products. The positive clones were sequenced by biotech Corporation. And the plasmid was named p-Anti-FAS-P18.

B. *In vitro* effective experiments

1. Cell line:

Hapatoma cell line HepG2 applied in the experiments was purchased from Shanghai Jinma Biological Technology Co., Ltd.

2. Major reagents

| Reagents | Source |
|---|---|
| RPMI1640 medium | Gibco |
| DMEM medium | Gibco |
| Lipofectamine 2000 | Invitrogen |
| Mycillin | Solarbio |
| Trypsin | BBI |
| Fetal bovine serum | Hyclone |

3. Dual-Luciferase reporter gene analysis

(1) Plated the cells at exponential phase (the cells in the above Table 1) at a density 0.75 × $10^5$ cells/ml in a 24-well plate with 500μl per well. Ideally cells should be 90% confluent prior to transfection;
(2) Reporter gene vectors containing promoter (SREBP-1c-571-Luc-WT, pFAS-WT-Luc, pGL3-NF-κB, 0.3μg each, purchased from Yuanhaoping (Tianjin) Biological Technology Co., Ltd) were transfected into cells by using lipofectamine transfection reagent, and Renilla luciferase expressing vectors (pRL-TK, 0.1μg, purchased from Promega) were transfected as controls. Simultaneously, different amount of polypeptides plasmids from Step A (0.25μg, 0.5μg and 0.75μg) or different concentrations of synthesized polypeptides from Example 1 (0.1μM, 1μM, 10μM and 100μM) were added, and repeated each concentration in three wells;
(3) After transfection for 48 hr (or after incubation with artificially synthesized polypeptide Anti-FAS-P 18 for 24 hr), washed the cells with PBS for three times;
(4) Added 100 μl 1x Passive Lysis Buffer (PLB) into transfected cells per well, and lysed the cell at room temperature for 15 min. Transferred the cell lysate to a 1.5 ml Eppendorf (EP) tube;
(5) Centrifuged at 12,000 rpm for 30 min, and transfered the supernatant to a fresh EP tube;
(6) Added an aliquot of cell lysate into each EP tube with 100 μl Luciferase Assay Buffer II (LARII), and mixed well;
(7) Immediately put the EP tubes into a Luminometer (manufactured by Turner Biosystems). Within 10 sec after 2 sec balance, measured the luminescence.
(8) Added 100 μl fluorescence bleacher. Firefly luciferase was quenched, while Renilla luciferase started to react.
(9) Measured the luminescence within 10 sec.

The relative activity was the ratio of the value of the first luminescence to the value of the second luminescence.
Results shown are representative of three independent experiments according to Mean±SD, and statistical significance was calculated using Student's *t* test.
As shown in the Figure 2, p-Anti-FAS-P18 inhibits the activities of SREBP-1c and FAS promoters in HepG2 cells in a dose-dependent manner, which indicates the expressed product has a function of inhibiting the transcription and expression of FAS gene. Student's *t*-test is employed for statistical analysis, * $P<0.05$, ** $P<0.01$.
As shown in the Figure 3, artificially synthesized polypeptide p-Anti-FAS-P18 inhibits the activities of SREBP-1c and FAS promoters in the HepG2 cells in a dose-dependent manner, which indicate the expressed product is capable of inhibiting the transcription and expression of FAS gene. Meanwhile, 100 μM variants of the invention polypeptides p-Anti-FAS-P18, including P18-1 to P18-5, also inhibit the activities of SREBP-1c and FAS promoters to different extents. Student's t-test is employed for statistical analysis, * $P<0.05$, ** $P<0.01$.

4. Immunoblotting Assay

1) Sample preparation

Collect the cells that are at a density of 90%, and wash with pre-cold PBS for 3 times;
Add 0.25% Tryspin to digest cells, and discard the Tryspin solution when cytoplasm retracts and cell interval increases, and then wash with pre-cold PBS for 3 times;
Add 10ml pre-cold PBS, suspends the cells, and transfer to centrifuge tube, 4000rpm/min , 4□, centrifuge for 10min and then collect the cells. Wash with pre-cold PBS for 2 times;
Completely remove the supernatant, add cell lysis buffer (8M urea, 4% CHAPS, 2% Pharmalyte 3-10) and add Protease Inhibitor Cocktail, incubate at room temperature for 30min;
13000rpm, 4□, centrifuge for 15min, collect the supernatant, and transfer to fresh Eppendorf tube, stored in -70□.

2) Protein concentration determination - Bradford Assay

Coomassie brilliant blue staining buffer contains 0.01%(W/V)G-250, 4.7%(W/V)ethanol, 8.5%(W/V) phosphate acid ;
Standard protein solution: bovine serum albumin (BSA), make up 2mg/ml protein solution with PBS buffer;
Set calibration curve: use 6 tubes, and operate as following table:

| Tube No. | 0 | 1 | 2 | 3 | 4 | 5 |
|---|---|---|---|---|---|---|
| PBS/μl | 100 | 90 | 80 | 70 | 60 | 50 |
| 2mg/ml BSA/μl | 0 | 10 | 20 | 30 | 40 | 50 |
| Coomassie brilliant blue staining buffer 3ml A595 nm | | | | | | |

Mix well, and sit for 10-15 min, colorimetric analyzed with Tube 0# as control at 595nm within 40 min. The calibration curve was plotted by A595nm as Y-axis, standard protein content as X-axis.

Measure protein concentration

Take another 4 tube, and operate as following table:

| Tube No. | 6 (SampleA) | 7(SampleA) | 8(SampleB) | 9 (SampleB) |
|---|---|---|---|---|
| PBS/μl | 95 | 90 | 95 | 90 |
| Sample/μl | 10 | 10 | 10 | 10 |
| Coomassie brilliant blue staining buffer 3ml A595nm | | | | |

Mix well, and sit for 10-15 min, colorimetric analyzed at 595nm.

As above-described detective method, collect proper volume of protein sample to make sure the estimated value within the region of calibration curve, and get the standard protein content on basis of estimated value, thereby calculating the protein concentration of sample.

3) The selection of resolving gel

| Molecular weight of Protein | Concentration of resolving gel |
|---|---|
| 60-200 kDa | 5% |
| 40-100 kDa | 8% |
| 16-70 kDa | 10% |
| 15-60 kDa | 12% |
| 12-45 kDa | 15% |

Prepare the resolving gel and stacking gel according to the following table, AP and TEMED were added in the end.

| Reagents | Resolving gel (ml) | | | | | Stacking gel (ml) |
|---|---|---|---|---|---|---|
| | 5% | 8% | 10% | 12% | 15% | 5% |
| 30% acrylamide | 1 | 2 | 3 | 4 | 5 | 0.83 |
| Resolving buffer (3M Tris-HCl) | 1.25 | 1.25 | 1.25 | 1.25 | 1.25 | - |
| Stacking buffer (0.5M Tris-HCl) | - | - | - | - | - | 1.26 |
| 10%SDS | 0.1 | 0.1 | 0.1 | 0.1 | 0.1 | 0.05 |
| ddH2O | 7.55 | 6.55 | 5.55 | 4.55 | 3.55 | 2.81 |
| 10%AP | 0.1 | 0.1 | 0.1 | 0.1 | 0.1 | 0.05 |
| 1%TEMED | 0.004 | 0.004 | 0.004 | 0.004 | 0.004 | 0.005 |

Pour resolving buffer to the mark, and add upper layer of water to the top of the gel to protect from air. Let polymerize, invert gel to remove water. Pour stacking buffer quickly, insert comb carefully to avoid bubbles. After polymerization, fill the running buffer, and remove the comb carefully. Add cell lysate solution and coomassie blue dye in the ratio of 4:1 to load samples, and run the SDS-PAGE (run at 100V through the stacking gel; run at 200V through the resolving gel).

4) Electrotransfer

Cut a piece of PVDF membrane and 6 pieces of filter paper to the size of the gel;
Immerse PVDF membrane in methanol for 10-15 sec, and then immerse in water for 2 min. Immerse PVDF membrane in transfer buffer for 15 min; immerse filter paper in transfer buffer;
On wet transfer electrophoresis tank, set up the transfer cassette from cathode-to-anode: (-) fiber pad - filter paper (two layer) - gel - PDVF membrane - filter paper - fiber pad (+); and roll out bubbles with glass tube. PVDF membrane closes to anode side, and keep wet. Run 90 min at 90V;
After transfer, clip one corner of the membrane as bottom left-band corner, and immerse in Ponceau's staining buffer for 5-10 min. After the protein bands appear, wash the membrane with ddH2O for several times;
Meanwhile, stain the gel in Coomassie brilliant blue staining buffer.

5) Immunoblotting

Blocking: soak PVDF membrane in 5% milk blocking buffer for 1h with shaking at room temperature or let it sit overnight at 4°C;
Add primary antibody (Rabbit anti-FAS antibody, purchased from Cell Signaling, USA). Prepare plastic bag, and put the membrane into it, and add proper diluted primary antibody onto the membrane (0.1ml/cm2). Roll out bubbles, and seal. Incubate the membrane for 2h with shaking at room temperature;
Wash. Wash the membrane with 0.1%Triton X-100 PBS buffer for 4 times, each for 15min;
Add secondary antibody labeled with horseradish peroxidase (broad-spectrum secondary antibody, purchased from Tianjing Jingmai Co.). Prepare plastic bag, and put the membrane into it, and add proper diluted secondary antibody onto the membrane (0.1ml/cm2). Roll out bubbles, and seal. Incubate the membrane for 2h with shaking at room temperature;
Wash. As step 3);
Add equivalent amount of ECLA, ECLB (purchased from Beijing Quanshijin Co.), mix well, and add the mixture onto the membrane to react 2-3 min;
Expose in dark room. Put plastic warp, membrane, plastic warp in order in the film container, and fix. Add film onto membrane in dark, and expose blot for 3-5 min;
After exposure, soak the film in developing solution until the bands appear, and then wash with water;
Soak the film in fixing bath for 3-5 min, and wash with water. Dry, and record.

As shown in Figure 4, p-Anti-FAS-P18 inhibits the expression level of FAS protein in HepG2 cells in a dose-dependent manner.
As shown in Figure 5, artificially synthesized polypeptide p-Anti-FAS-P18 inhibits the expression level of FAS protein in HepG2 cells in a dose-dependent manner.

5. MTT assay

(1) Plate the cells: suspended the cells at exponential phase (the cells in the above Table 1) in RPMI1640 or DMEM medium with 10% FBS, and plated 4000-5000 cells in a 96-well plate with 100μl per well;
(2) Culture the cells: Within 12 hr for adhesion of cultured cells, different amount of polypeptides plasmids from Step A (0.25μg, 0.5μg and 0.75μg) or different concentrations of synthesized polypeptides from Example 1 (0.1μM, 1μM, 10μM and 100μM) were transfected with 8 wells for each concentration, and were incubated for 48 hr in the same condition. 0.3μg pEGFP-C2 plasmids and 1μg p-Anti-HBxP1# were cotransfected, and using a fluorescence microscope to check for cells containing the GFP plasmid after 24 hr to make sure the percentage of cells that contain GFP is over 70%;
(3) Coloration: added 20μl MTT solution (5 mg/ml MTT in PBS buffer (pH7.4)) for each well;
(4) Incubated for 4 hr, and carefully aspirate off the supernatant from the wells. Added 150μl DMSO into each well, shaking for 10 min until the crystals dissolved;
(5) Comparison: Read in an ELISA reader at 490 nm to measure the absorbance for each well. Results were calculated by using Student's t-test.

As shown in the Figure 6, p-Anti-FAS-P18 inhibits the activity of NF-κB promoter in HepG2 cells in a dose-dependent manner, which indicates the capacity of cell proliferation of hepatoma cells decreases. Student's t-test is employed for statistical analysis, * P<0.05, ** P<0.01.

As shown in the Figure 7, Anti-FAS-P18 inhibits the activity of NF-κB promoter in HepG2 cells in a dose-dependent manner, which indicate the capacity of cell proliferation of hepatoma cells decreases. 100 μM variants of polypeptides Anti-FAS-P18 in the present invention, including P18-1 to P18-5, also inhibit the activity of NF-κB promoter to different extent. Student's *t*-test is employed for statistical analysis, * P<0.05, ** P<0.01.

Example 3: The anti-FAS activities of polypeptides *in vivo* Suspended the HepG2cells at exponential phase by treating with trypsin, and counted the number of cells to dilute to 1 x $10^7$ cells/ml with physiological saline, and then store in the ice water. 12 Mice used were 4-to 6-week-old BALB/C females, and were randomly divided into two groups: ① Control group, injected 0.2ml diluted cells to the armpit of left forelimb for each mouse, and only injected 0.5ml $ddH_2O$ (without polypeptide drugs); ② experimental group (the treatment dose was 10 mg/kg), injected 0.2ml diluted cells to the armpit of left forelimb for each mouse, and within 7 days after the injection, tumor volume ($V=L\times W^2\times 0.5$) reached to 100 $mm^3$. And then, injected the above mentioned polypeptide drugs (dried polypeptide drug solved in 0.5ml $ddH_2O$) once in two day, total 10 times, and recorded the tumor volume before injection. Within 24 hr after the last dose, weighted the mice, and killed the mice to weight the tumor tissue, anti-tumor rate was calculated as follow:

$$\text{anti-tumor rate}=\frac{\text{The average tumor volume of control- experiment group}}{\text{The average tumor volume of control}}\times 100\%$$

As shown in Table 1 and Figure 10, the results of animal experiment indicate that artificially synthesized polypeptide Anti-FAS-P 18 inhibits the growth and proliferation in the HepG2 cells. Student's t-test was employed for statistical analysis, ** P<0.01.

As shown in Figure 11, the results of inoculation experiment in nude mice shows that Anti-FAS-P18 has no significantly impact on the weight of nude mice. Student's t-test was employed for statistical analysis.

Table 1. The effect of artificially synthesized polypeptide Anti-FAS-P18 in the HepG2 cells:

| Group | Before dose/After dose | Weight (X±S, g) | | Tumor weight (X±S, g) | Anti-tumor rate (%) |
|---|---|---|---|---|---|
| | | Before dose | After dose | | |
| Control | 6/6 | 15.58±0.97 | 22.08±1.02 | 0.73±0.11 | - |
| Experimental | 6/6 | 15.33±0.75 | 21.83±0.82 | 0.42±0.09 | 43.06 |

Example 4: The functional fragments and functional variants

The invention also provides the role of the functional fragments and functional variants. The sequences in the table below are obtained by adding amino acids (e.g., add an amino acid in the terminus) or replacing a conserved amino acid (e.g., replace an amino acid with the same type of an amino acid) on basis of Anti-FAS-P18. Artificially synthesized the polypeptide fragments according to the sequences (method as above), and then, used the above-mentioned gene reporter and MTT assay, and observe the change of the role of the polypeptides.

| Synthetic polypeptide fragments | Amino acid sequence | SEQ ID NO. |
|---|---|---|
| Anti-FAS-P18 (P18) | Gly–Gly-Cys-Arg-His–Lys-Leu-Val-Cys-Ala-Pro-Ala-Pro-Cys-Asn-Phe-Phe-Thr<br>或 G-G-C-R-H-K-L-V-C-A-P-A-P-C-N-F-F-T | 1 |
| Anti-FAS-P18-1 (P18-1) | Val-Gly-Gly-Cys-Arg-His-Lys-Leu-Val-Cys-Ala-Pro-AlaPro-Cys-Asn-Phe-Phe-Thr<br>或 V-G-G-C-R-H-K-L-V-C-A-P-A-P-C-N-F-F-T | 2 |

(continued)

| Synthetic polypeptide fragments | Amino acid sequence | SEQ ID NO. |
|---|---|---|
| Anti-FAS-P18-2 (P18-2) | Ile-Gly-Gly-Cys-Arg-His-Lys-Leu-Val-Cys-Ala-Pro-Ala-Pro-Cys-Asn-Phe-Phe-Thr<br>或 I-G-G-C-R-H-K-L-V-C-A-P-A-P-C-N-F-F-T | 3 |
| Anti-FAS-P18-3 (P18-3) | Gly-Gly-Cys-Lys-His-Lys-Leu-Val-Cys-Ala-Pro-Ala-Pro-Cys-Asn-Phe-Phe-Thr<br>或 G-G-C-K-H-K-L-V-C-A-P-A-P-C-N-F-F-T | 4 |
| Anti-FAS-P18-4 (P18-4) | Gly-Gly-Arg-Arg-His-Lys–Leu-Val-Cys-Ala-Pro-Ala-Pro-Cys-Asn-Phe-Phe-Thr<br>或 G-G-C-R-R-K-L-V-C-A-P-A-P-C-N-F-F-T | 5 |
| Anti-FAS-P18-5 (P18-5) | Gly-Gly-Cys-Arg-His-His-Leu-Val-Cys-Ala-Pro-Ala-Pro-Cys-Asn-Phe-Phe-Thr<br>或 G-G-C-R-H-H-L-V-C-A-P-A-P-C-N-F-F-T | 6 |
| Synthetic polypeptide fragments | Nucleotide sequence | SEQ ID NO. |
| Anti-FAS-P18 (P18) | ggaggctgta ggcataaatt ggtctgcgca ccagcaccat gcaacttttt cacc | 7 |
| Anti-FAS-P 18-1 (P18-1) | gtcggaggct gtaggcataa attggtctgc gcaccagcac catgcaactt tttcacc | 8 |
| Anti-FAS-P18-2 (P18-2) | atcggaggct gtaggcataa attggtctgc gcaccagcac catgcaactt tttcacc | 9 |
| Anti-FAS-P18-3 (P18-3) | ggaggctgta aacataaatt ggtctgcgca ccagcaccat gcaacttttt cacc | 10 |
| Anti-FAS-P18-4 (P18-4) | ggaggctgtc atcataaatt ggtctgcgca ccagcaccat gcaacttttt cacc | 11 |
| Anti-FAS-P18-5 (P18-5) | ggaggctgta ggcatcattt ggtctgcgca ccagcaccat gcaacttttt cacc | 12 |

As shown in the Figure 3, Figure 7 and Figure 9, the variants of invention polypeptide Anti-FAS-P18 inhibit the activity of SERBP-1c, FAS and NF-κB promoter and cell proliferation of the HepG2 cells to different extend. Student's t-test was employed for statistical analysis, * $P<0.05$, ** $P<0.01$.

Example 5: Polypeptide drugs acute toxicity test

Control group and experimental group contained 10 Kunming white mice, with 5 females and 5 males, respectively. Experimental group injected Anti-FAS-P18 with the concentration of 1g/kg (2.5mg dried polypeptide drug solved in 0.125ml $ddH_2O$) by tail intravenous injection; control group, injected 0.25ml $ddH_2O$. Consecutively observed the mice for 24 hr after injection.

The polypeptide drugs acute toxicity test showed that the mice do not have abnormal behaviors, and no abnormal changes in weight compared with control group.

All references, including publications, patent applications, and patents, cited herein are hereby incorporated and were set forth in its entirety herein. The use of any and all examples, or exemplary language (e.g., "such as") provided herein, is intended merely to better illuminate the invention and does not pose a limitation on the scope of the invention unless otherwise claimed. Preferred embodiments of this invention are described herein, including the best mode known to the inventors for carrying out the invention. Variations of those preferred embodiments may become apparent to those of ordinary skill in the art upon reading the foregoing description. The inventors expect skilled artisans to employ such variations as appropriate, and the inventors intend for the invention to be practiced otherwise than as specifically described herein. The invention includes all modifications and equivalents of the subject matter recited in the claims appended hereto as permitted by applicable law unless otherwise indicated herein or otherwise clearly contradicted by context.

## Claims

1. An isolated polypeptide or peptidomimetic, comprising amino acid sequence as shown in SEQ ID NO: 1, or functional fragment or variant thereof, said polypeptide or peptidomimetic having an effect of inhibiting anti-fatty acid synthase and the occurrence and development of tumors, fatty liver and obesity.

2. A polypeptide or peptidomimetic according to claim 1, wherein said tumors comprise liver cancer.

3. A polypeptide or peptidomimetic according to claim 1, comprising amino acid sequence that has at least 80% identity with the amino acid sequence as shown in SEQ ID NO: 1.

4. A polypeptide or peptidomimetic according to claim 1, comprising amino acid sequence that has at least 90% identity with the amino acid sequence as shown in SEQ ID NO: 1.

5. A polypeptide or peptidomimetic according to claim 1, comprising amino acid sequence that has at least 95% identity with the amino acid sequence as shown in SEQ ID NO: 1.

6. A polypeptide or peptidomimetic according to claim 1, comprising amino acid sequence obtained from conservative substitution of one amino acid of SEQ ID NO:1, or addition or deletion of one amino acid at the terminus of of SEQ ID NO: 1.

7. A polypeptide or peptidomimetic according to claim 1, comprising any one of the amino acid sequences as shown in SEQ ID NOs: 1-6.

8. An isolated polynucleotide, said polynucleotide is selected from the group consisting of:

    a) the polynucleotide encoding the amino acid sequence as shown in SEQ ID NO: 1 or the functional fragment or functional variant thereof; and
    b) a polynucleotide that is complementary to the polynucleotide of a) or that is capable of hybridizing with the polynucleotide of a) under stringent condition.

9. A polynucleotide according to claim 8, said polynucleotide is selected from the group consisting of:

    a) the polynucleotide encoding any one of the amino acid sequences as shown in SEQ ID NOs: 1-6.; and
    b) a polynucleotide that is complementary to the polynucleotide of a) or that is capable of hybridizing with the polynucleotide of a) under stringent condition.

10. A recombination expression vector comprising the polynucleotide of claim 8 or 9.

11. A host cell including the recombination expression vector of claim 10.

12. The use of the polypeptide according to any one of claims 1-7 in manufacturing medicines for treating and preventing tumors, fatty liver and obesity.

13. The use of the polynucleotide according to any one of claims 8-9 in manufacture of medicines for treating and preventing tumors, fatty liver and obesity.

14. The use of the recombination expression vector according to claim 10 in manufacture of medicines for treating and preventing tumors, fatty liver and obesity.

15. A pharmaceutical composition, comprising any one of the polypeptides or peptidomimetic according to claims 1-7,

and optional pharmaceutical carriers.

**16.** A pharmaceutical composition, comprising any one of the polynucleotides according to any one of claims 8-9, and optional pharmaceutical carriers.

**17.** A pharmaceutical composition, comprising the recombination expression vector according to claim 10, and optional pharmaceutical carriers.

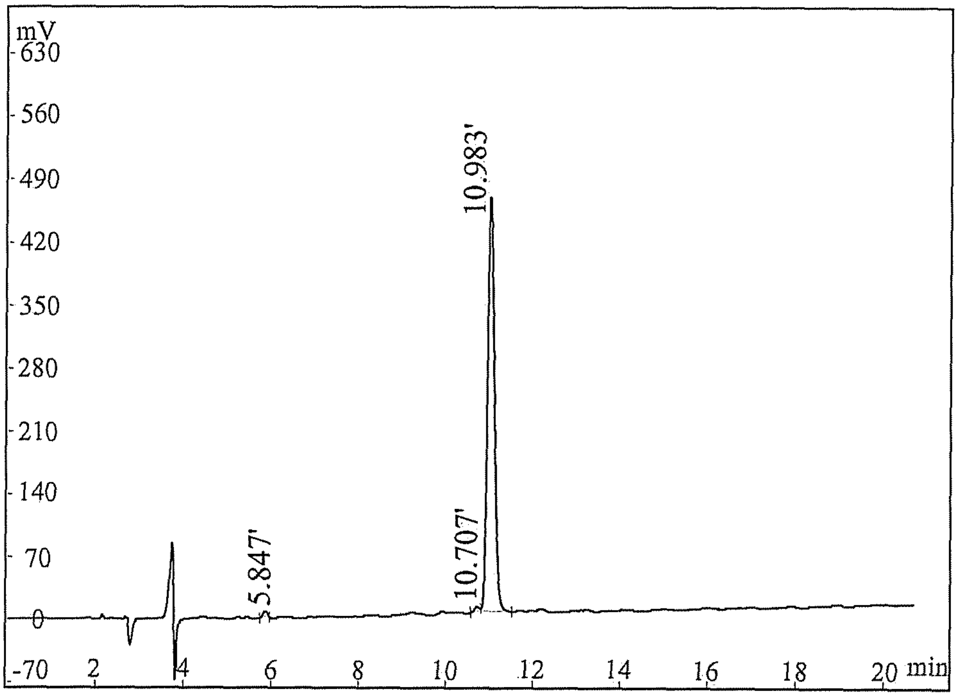
mV
630
560
490
420
350
280
210
140
70
0
-70
2
4
6
8
10
12
14
16
18
20
min
10.983'
10.707'
5.847'

Fig.1

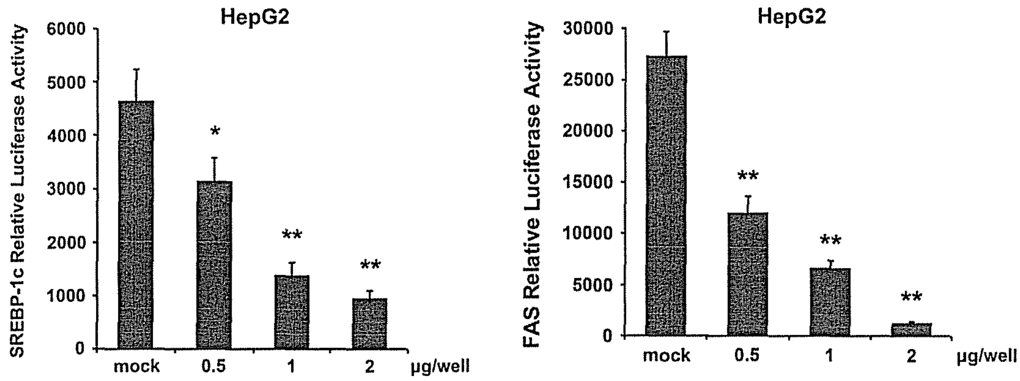
HepG2
SREBP-1c Relative Luciferase Activity
6000
5000
4000
3000
2000
1000
0
*
**
**
mock
0.5
1
2
µg/well
HepG2
FAS Relative Luciferase Activity
30000
25000
20000
15000
10000
5000
0
**
**
**
mock
0.5
1
2
µg/well

Fig.2

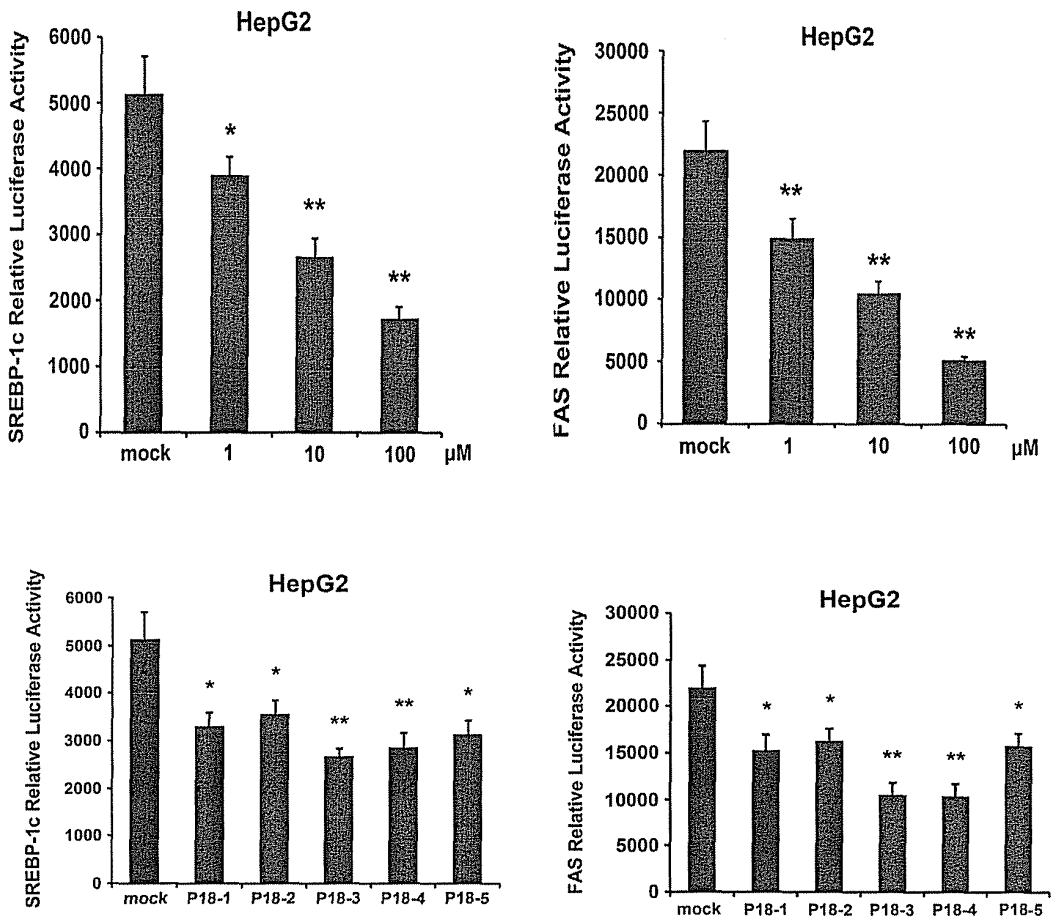
HepG2
SREBP-1c Relative Luciferase Activity
6000
5000
4000
3000
2000
1000
0
*
**
**
mock
1
10
100
µM
HepG2
FAS Relative Luciferase Activity
30000
25000
20000
15000
10000
5000
0
**
**
**
mock
1
10
100
µM
HepG2
SREBP-1c Relative Luciferase Activity
6000
5000
4000
3000
2000
1000
0
*
*
**
**
*
mock
P18-1
P18-2
P18-3
P18-4
P18-5
HepG2
FAS Relative Luciferase Activity
30000
25000
20000
15000
10000
5000
0
*
*
**
**
*
mock
P18-1
P18-2
P18-3
P18-4
P18-5

Fig.3

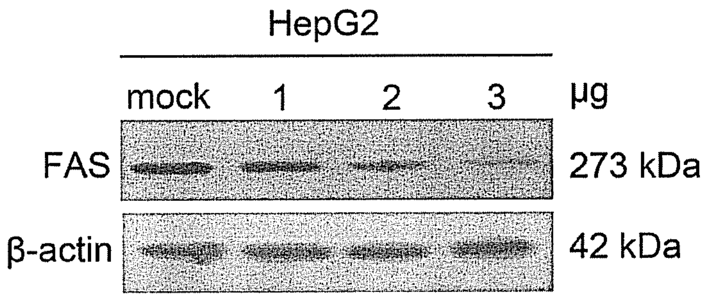


Fig.4

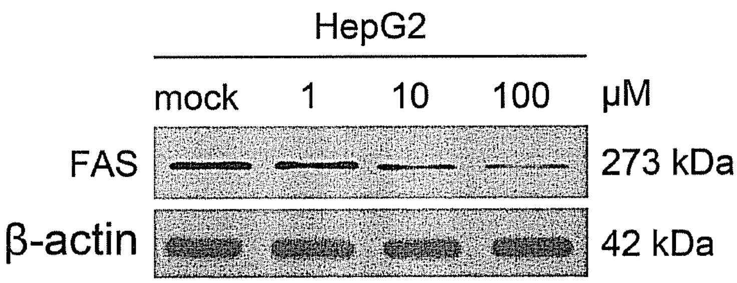


Fig.5

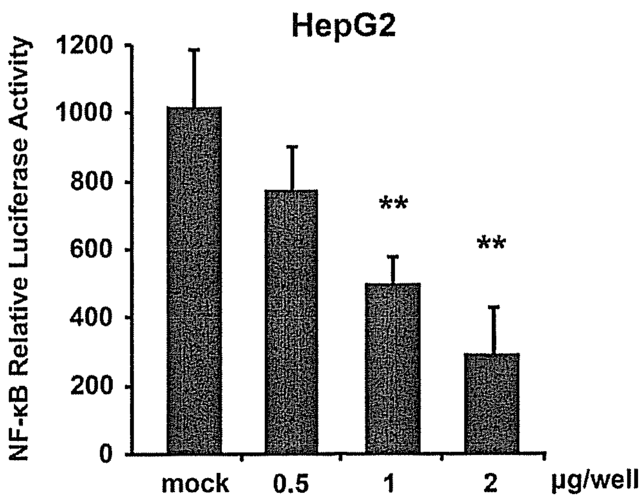


Fig.6

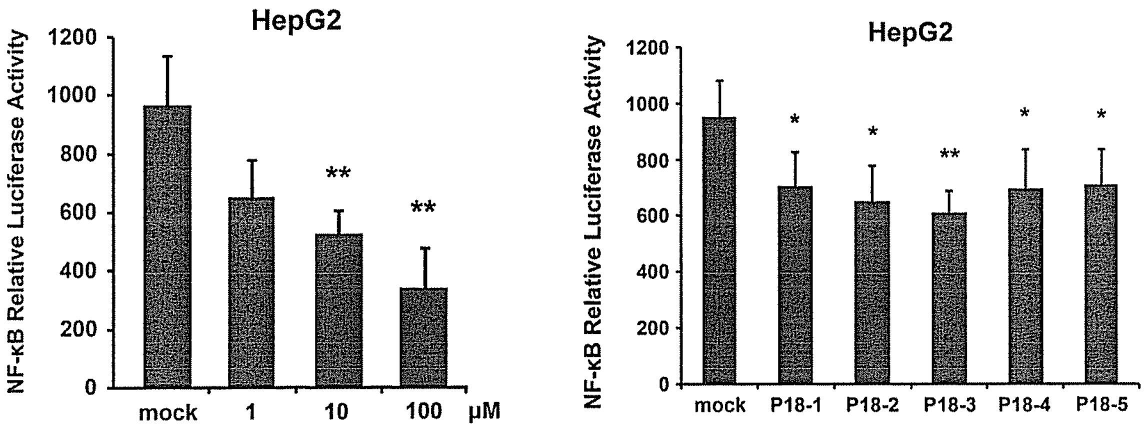
HepG2
NF-κB Relative Luciferase Activity
1200
1000
800
600
400
200
0
**
**
mock
1
10
100
μM
HepG2
NF-κB Relative Luciferase Activity
1200
1000
800
600
400
200
0
*
*
**
*
*
mock
P18-1
P18-2
P18-3
P18-4
P18-5

Fig.7

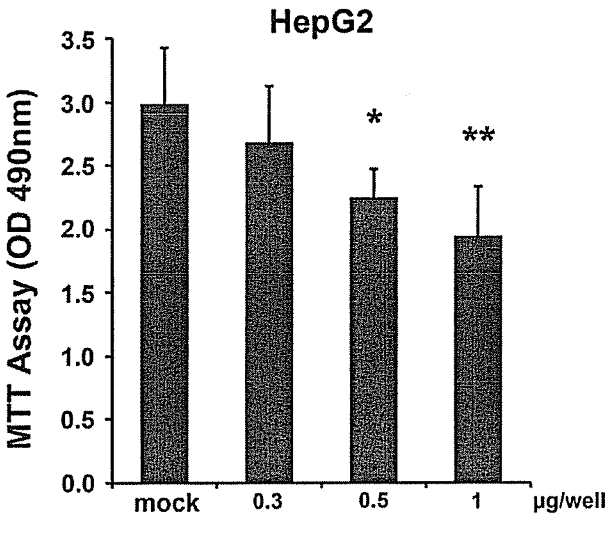
HepG2
MTT Assay (OD 490nm)
3.5
3.0
2.5
2.0
1.5
1.0
0.5
0.0
*
**
mock
0.3
0.5
1
μg/well

Fig.8

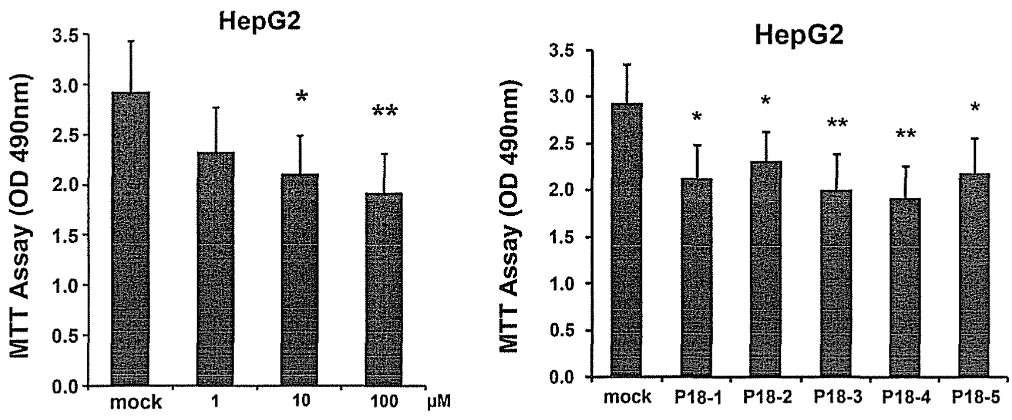
HepG2
MTT Assay (OD 490nm)
3.5
3.0
2.5
2.0
1.5
1.0
0.5
0.0
*
**
mock
1
10
100
μM
HepG2
MTT Assay (OD 490nm)
3.5
3.0
2.5
2.0
1.5
1.0
0.5
0.0
*
*
**
**
*
mock
P18-1
P18-2
P18-3
P18-4
P18-5

Fig.9

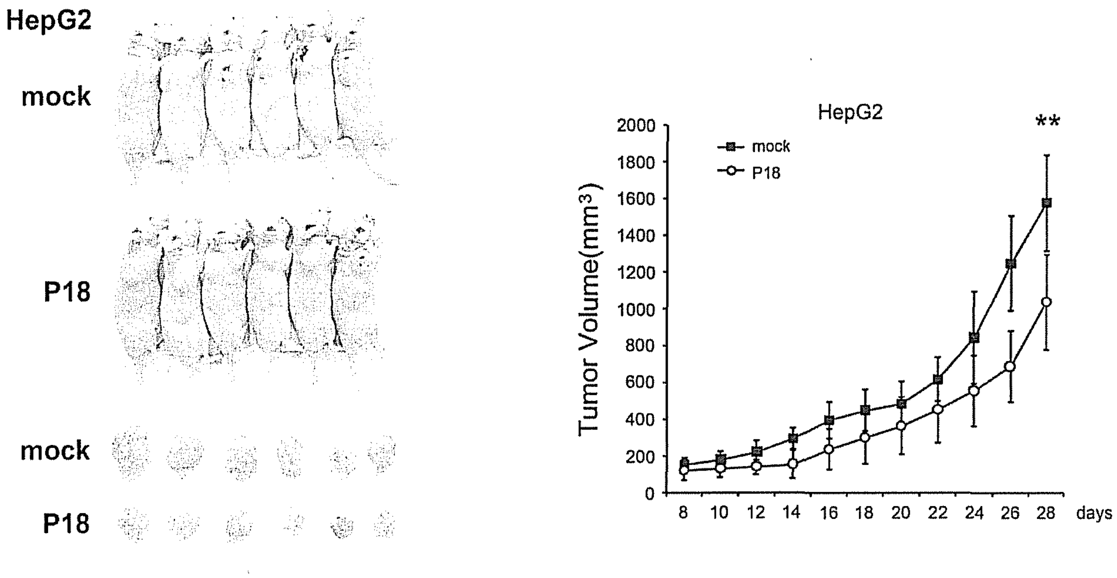
HepG2
mock
P18
mock
P18
HepG2
**
mock
P18
Tumor Volume(mm³)
2000
1800
1600
1400
1200
1000
800
600
400
200
0
8
10
12
14
16
18
20
22
24
26
28
days

Fig.10

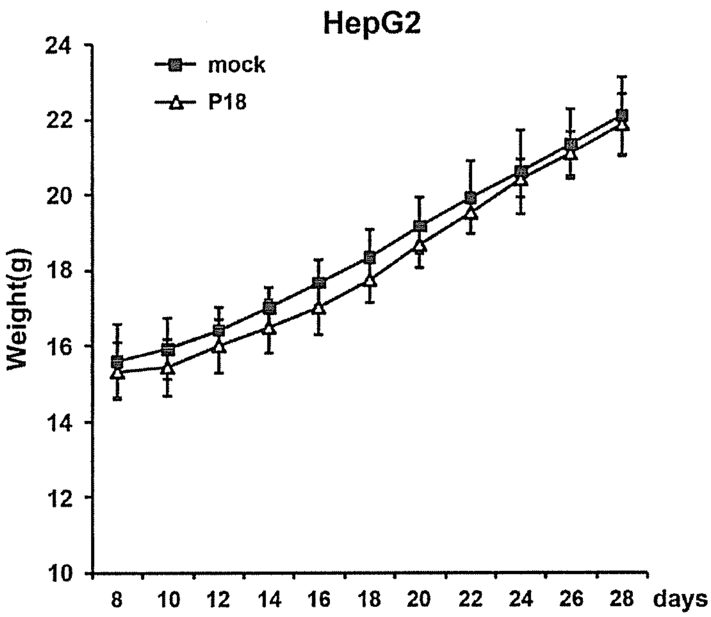
HepG2
mock
P18
Weight(g)
24
22
20
18
16
14
12
10
8
10
12
14
16
18
20
22
24
26
28
days

Fig.11

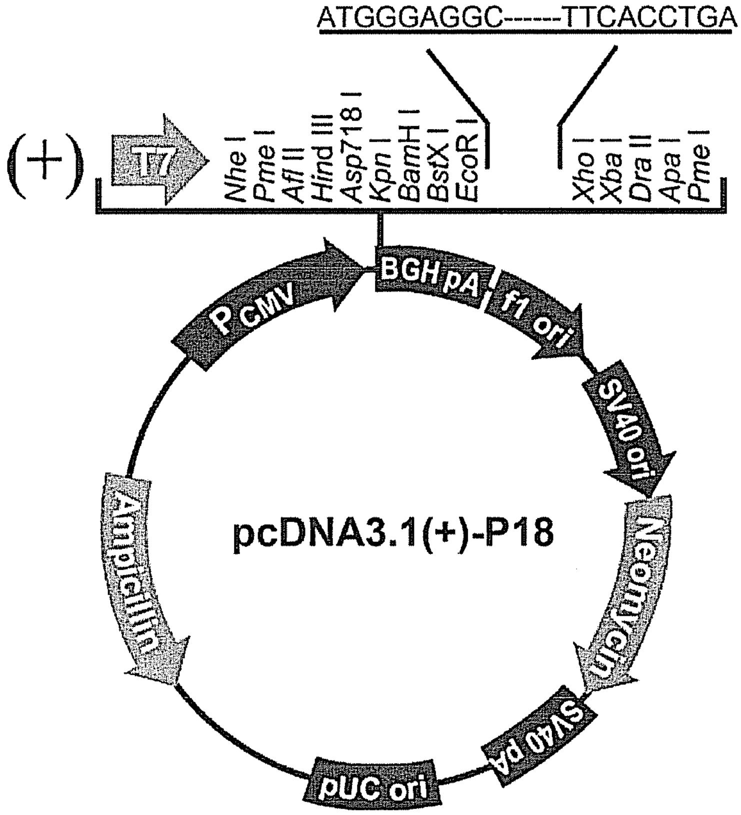
ATGGGAGGC------TTCACCTGA
(+)
T7
Nhe I
Pme I
Afl II
Hind III
Asp718 I
Kpn I
BamH I
BstX I
EcoR I
Xho I
Xba I
Dra II
Apa I
Pme I
P CMV
BGH pA
f1 ori
SV40 ori
Neomycin
SV40 pA
pUC ori
Ampicillin
pcDNA3.1(+)-P18

Fig.12

# INTERNATIONAL SEARCH REPORT

International application No.
**PCT/CN2012/074107**

**A. CLASSIFICATION OF SUBJECT MATTER**

see the extra sheet

According to International Patent Classification (IPC) or to both national classification and IPC

B. FIELDS SEARCHED

Minimum documentation searched (classification system followed by classification symbols)

IPC: C07K; C12N; A61K; A61P

Documentation searched other than minimum documentation to the extent that such documents are included in the fields searched

Electronic data base consulted during the international search (name of data base and, where practicable, search terms used)

DWPI; SIPOABS; BIOSIS; MEDLINE; Google Scholar; CNABS; CPRS; CNKI and keywords: fatty acid synthase, FAS, inhibit+, hepatitis, peptide?, protein X, obesity, adiposity, liver cancer, fatty liver etc.
EMBL; GenBank: SEQ ID NOs :1-12

C. DOCUMENTS CONSIDERED TO BE RELEVANT

| Category* | Citation of document, with indication, where appropriate, of the relevant passages | Relevant to claim No. |
|---|---|---|
| X | CN101502642 A(UNIV SICHUAN) 12 Aug. 2009(12. 08. 2009) claims 1-7, description, page 2, embodiments 1-3 and SEQ ID NOs :1-2 | 1-17 |
| X | EMBL accession number:C7DNM6，18 May 2010(18.05.2010) see the amino acid sequence | 1-11, 15-17 |
| X | KIM, C. et al., Nucleotide Sequence Determination and Expression of X OpenReading Frameof a Hepatitis B Virus adr Subtype, Korean Biochemical Journal , 1988, vol. 21, No.4, pages 332-338 | 1-11, 15-17 |

☒ Further documents are listed in the continuation of Box C. ☒ See patent family annex.

* Special categories of cited documents:

"A" document defining the general state of the art which is not considered to be of particular relevance

"E" earlier application or patent but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&"document member of the same patent family

| Date of the actual completion of the international search | Date of mailing of the international search report |
|---|---|
| 09 Jul. 2012 (09. 07. 2012) | 26 Jul. 2012 (26. 07. 2012) |

Name and mailing address of the ISA
State Intellectual Property Office of the P. R. China
No. 6, Xitucheng Road, Jimenqiao
Haidian District, Beijing 100088, China
Facsimile No. (86-10)62019451

Authorized officer
WANG, Boli
Telephone No. **62411994**

Form PCT/ISA /210 (second sheet) (July 2009)

| INTERNATIONAL SEARCH REPORT | International application No. PCT/CN2012/074107 |
|---|---|

C (Continuation). DOCUMENTS CONSIDERED TO BE RELEVANT

| Category* | Citation of document, with indication, where appropriate, of the relevant passages | Relevant to claim No. |
|---|---|---|
| X | WO2010118324A2 (NUCLEA BIOTECHNOLOGIES INC) 14. Oct. 2010 (14.10. 2010), claims 1-2 and 8-10, description, embodiments 1-3 and abstract | 1-2, 8, 10-17 |
| A | CN 101284000 A(UNIV SICHUAN) 15. Oct. 2008(15. 10. 2008) the whole document | 1-17 |
| A | CN 101768081 A(UNIV CAPITAL MEDICAL SCI ) 07 Jul. 2010(07. 07. 2010) the whole document | 1-17 |

Form PCT/ISA /210 (continuation of second sheet ) (July 2009)

**INTERNATIONAL SEARCH REPORT**
Information on patent family members

International application No.
**PCT/CN2012/074107**

| Patent Documents referred in the Report | Publication Date | Patent Family | Publication Date |
|---|---|---|---|
| CN101502642 A | 12.08.2009 | NONE | |
| WO2010118324A2 | 14.10.2010 | WO2010118324A3<br>US2012020978A1 | 02.12.2010<br>26.01.2012 |
| CN 101284000 A | 15. 10. 2008 | NONE | |
| CN 101768081 A | 07. 07. 2010 | NONE | |

Form PCT/ISA /210 (patent family annex) (July 2009)

**INTERNATIONAL SEARCH REPORT**

International application No.
**PCT/CN2012/074107**

Continuation of : CLASSIFICATION OF SUBJECT MATTER

C07K 7/08 (2006.01) i

C12N 15/11 (2006.01) i

C12N 15/63 (2006.01) i

A61K 38/ 10(2006.01) i

A61P 35/ 00(2006.01) i

A61P 1/ 16(2006.01) i

A61P 3/ 04(2006.01) i

Form PCT/ISA /210 (extra sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 201110102020
- US 5449752 A
- US 5087616 A
- US 4450150 A


### Non-patent literature cited in the description


- **SAMBROOK et al.** Molecular Cloning: A Laboratory Manual. Cold Spring Harbor Laboratory, 2001
- **HIGGINS ; SHARP.** *Gene,* 1988, vol. 73, 237-244
- **HEIN J.** *the Methods in Emzumology,* 1990, vol. 183, 625-645
- **CHAN et al.** Fmoc Solid Phase Peptide Synthesis. Oxford University Press, 2005
- **REID, R.** Peptide and Protein Drug Analysis. Marcel Dekker Company, 2000
- **SAMBROOK et al.** Molecular Cloning: A Laboratory Manual. Cold Spring Harbor Press, 2001
- Cancer Research UK Centre for Cancer Therapeutics at the Institute of Cancer Research. **CAROLINE J.** Suicide Gene Therapy: Methods and Reviews. Springer, 2004
- **HUDECZ, F.** *Methods Mol Biol.,* 2005, vol. 298, 209-223
- **KIRIN et al.** *Inorg Chem,* 2005, vol. 44 (15), 5405-5415
- **WADWA et al.** *J. Drug Targeting,* 1995, vol. 3, 111